(19) Europäisches Patentamt
European Patent Office
Office européen des brevets

(11) Numéro de publication: **0 480 795 B1**

(12) **FASCICULE DE BREVET EUROPEEN**

(45) Date de publication de fascicule du brevet: **07.09.94**

(51) Int. Cl.5: **C07D 307/79**, C07D 307/80, A01N 43/12

(21) Numéro de dépôt: **91402618.2**

(22) Date de dépôt: **01.10.91**

# (54) Nouveaux dérivés du benzofuranne, leur procédé de préparation, les nouveaux intermédiaires obtenus et leur application comme pesticides.

(30) Priorité: **02.10.90 FR 9012103**

(43) Date de publication de la demande: **15.04.92 Bulletin 92/16**

(45) Mention de la délivrance du brevet: **07.09.94 Bulletin 94/36**

(84) Etats contractants désignés: **CH DE ES FR GB IT LI NL**

(56) Documents cités:
**EP-A- 0 243 014**
**US-A- 4 143 154**

(73) Titulaire: **ROUSSEL-UCLAF**
**35, Boulevard des Invalides**
**F-75007 Paris (FR)**

(72) Inventeur: **Benoit, Marc**
**Le Cannet Est,**
**Pont de l'Etoile**
**F-13360 Roquevaire (FR)**
Inventeur: **Brayer, Jean-Louis**
**42, rue Jules Dubrulle**
**F-60440 Nanteuil le Haudoin (FR)**
Inventeur: **Demoute, Jean-Pierre**
**65, Avenue Foch**
**F-93360 Neuilly Plaisance (FR)**
Inventeur: **Mourioux, Gilles**
**12, Allée des Micocouliers**
**F-13420 Gemenos (FR)**
Inventeur: **Taliani, Laurent**
**107, Allée Danielle Casanova**
**F-93320 Pavillons sous Bois (FR)**

(74) Mandataire: **Tonnellier, Marie-José et al**
**111, route de Noisy**
**B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne de nouveaux dérivés du benzofuranne, leur procédé de préparation , les nouveaux intermédiaires obtenus et leur application comme pesticides.

L'invention a pour objet les composés de formule (I) :

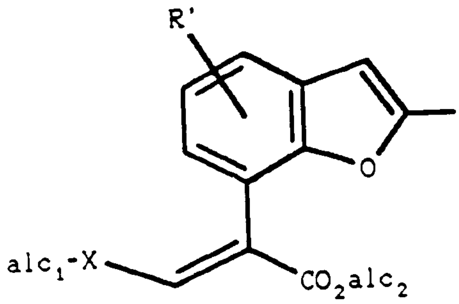

(I)

dans laquelle :

- R représente un atome d'hydrogène, un radical alkyle, alcényle ou alcynyle renfermant jusqu'à 8 atomes de carbone éventuellement substitué, un radical cycloalkyle ou cycloalcényle renfermant jusqu'à 8 atomes de carbone éventuellement substitué, un radical aryle renfermant jusqu'à 18 atomes de carbone éventuellement substitué, un radical arylalkyle renfermant jusqu'à 24 atomes de carbone éventuellement substitué, un radical benzoyle éventuellement substitué ou un radical hétérocyclique éventuellement substitué,
- $alc_1$ et $alc_2$ identiques ou différents représentent un radical alkyle renfermant jusqu'à 8 atomes de carbone éventuellement substitué,
- X représente un atome d'oxygène ou de soufre,
- R′ en position quelconque sur le noyau phényle représente un atome d'hydrogène ou un atome d'halogène,

  la géométrie de la double liaison étant E ou Z ou un mélange E et Z.

Lorsque R représente un radical alkyle, il s'agit de préférence d'un radical méthyle, éthyle, propyle, isopropyle, butyle, isobutyle, sec-butyle, tert-butyle, pentyle ou hexyle.

Lorsque R représente un radical alcényle, il s'agit de préférence d'un radical vinyle ou 1,1-diméthyl allyle.

Lorsque R représente un radical alcynyle, il s'agit de préférence d'un radical éthynyle ou propynyle.

Lorsque R représente un radical cycloalkyle, il s'agit de préférence du radical cyclohexyle.

Lorsque R représente un radical cycloalcényle, il s'agit de préférence du radical cyclohexényle.

Lorsque R représente un radical alkyle, alcényle, alcynyle, cycloalkyle ou cycloalkényle substitué, il s'agit de préférence d'un radical alkyle, alcényle ou alcynyle substitué par un ou plusieurs atomes d'halogène par exemple d'un radical $-CH_2F$, $CHF_2$, $CF_3$, $CCl_3$, $CBr_3$.

Lorsque R représente un radical aryle, il s'agit de préférence d'un radical phényle, biphényle ou naphtyle.

Lorsque R représente un radical arylalkyle, il s'agit de préférence d'un radical benzyle, éventuellement substitué sur le radical méthylène par un radical hydroxy ou hydroxyimino.

Lorsque R représente un radical hétérocyclique, il s'agit de préférence d'un radical hétérocyclique renfermant 1 ou plusieurs atomes d'azote, d'oxygène, ou de soufre, par exemple un radical choisi dans le groupe constitué par les radicaux suivants : thiényle, furyle, pyrrolyle, imidazolyle, pyrazolyle, pyridyle, pyrazinyle, pyrimidinyle, pyridazinyle, oxazylyle, isothiazolyle, isoxazolyle, thiazolyle, oxadiazolyle, thiadiazolyle.

Lorsque R représente un radical aryle, arylalkyle, benzoyle ou hétérocyclique substitué, de préférence choisis dans le groupe constitué par les atomes d'halogènes, les radicaux alkyle renfermant jusqu'à 8 atomes de carbone, $CF_3$, $CH_2F$, $CHF_2$, CN, $NO_2$, alcoxycarbonyle, $CONH_2$, $OCF_3$, phényle ou phénoxy éventuellement substitué, alkylamino ou dialkylamino renfermant de 1 à 4 atomes de carbone, il peut s'agir de deux substituants, portés par 2 carbonates adjacents, formant un radical alkylène dioxy, ou de un ou plusieurs substituants, OR″, SR″ dans lesquels R″ représente un radical alkyle ou alcényle renfermant jusqu'à 6 atomes de carbone, ou les radicaux hétérocycliques : thiényle, furyle, thiazolyle, oxazolyle, pyridinyle, pyrazinyle, pyrimidinyle, isoxazolyle, isothiazolyle, oxadiazolyle, thiadiazolyle tels que définis ci-dessus.

Par alkylamino ou dialkylamino, on entend par exemple méthylamino, éthylamino, diméthylamino, diéthylamino, méthyléthylamino.

L'invention a plus particulièrement pour objet les composés de formule (I) dans lesquels X représente un atome d'oxygène, les composés de formule (I) dans lesquels R' représente un atome d'hydrogène, les composés de formule (I) dans lesquels $alc_1$ et $alc_2$ représentent chacun un radical méthyle, les composés de formule (I) dans lesquels R représente un radical alkyle renfermant jusqu'à 8 atomes de carbone, notamment un radical n-butyle, les composés de formule (I) dans lesquels R représente un radical phényle éventuellement substitué par un ou plusieurs radicaux choisis dans le groupe constitué par les atomes d'halogène, notamment le chlore et le brome, les radicaux alkyloxy renfermant jusqu'à 6 atomes de carbone, de préférence jusqu'à 4 atomes de carbone notamment le radical méthoxy, les radicaux phénoxy, thiazolyle et alkyle substitué par un ou plusieurs atomes d'halogène, notamment le radical trifluorométhyle ou R représente un radical phényle substitué sur 2 carbones adjacents par un radical alkylènedioxy, notamment le radical 3,4-méthylènedioxyphényle, ainsi que les composés de formule (I) dans lesquels R représente un radical thiazolyle éventuellement substitué par un radical alkyle linéaire ou ramifié renfermant jusqu'à 8 atomes de carbone, de préférence jusqu'à 4 atomes de carbone et notamment le radical isopropyle, ou par un radical phényle éventuellement substitué, notamment le radical trifluorométhoxyphényle.

L'invention a plus particulièrement pour objet les composés de formule (I) dans lesquels la double liaison éther d'énol est de géométrie E.

L'invention a tout spécialement pour objet les composés de formule (I) dont la préparation est donnée ci-après dans la partie expérimentale notamment les composés des exemples 1, 3, 11, 14, 15, 16, 17, 18, 19 et 28.

L'invention a également pour objet un procédé de préparation des composés de formule (I) caractérisé en ce que l'on soumet un composé de formule (II) :

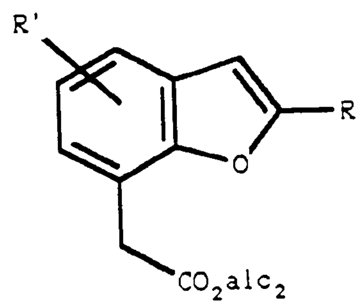

(II)

dans laquelle R, R' et $alc_2$ conservent leur signification précédente à l'action d'un agent de formylation pour obtenir le composé de formule (III) :

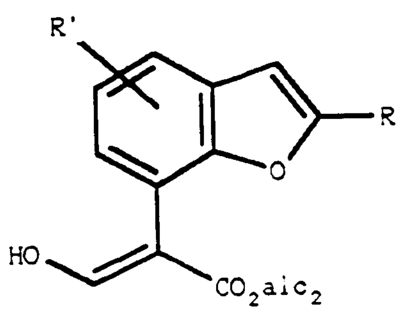

(III)

que l'on soumet à l'action d'un agent d'alkylation pour obtenir le composé de formule ($I_A$) :

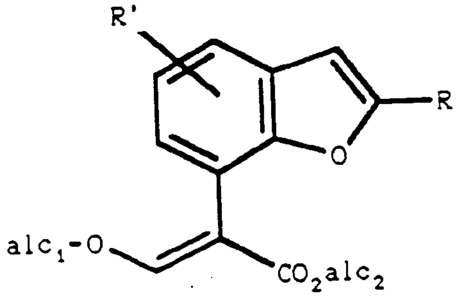

($I_A$)

dans laquelle $alc_1$ conserve sa signification précédente que l'on soumet si désiré à l'action d'un agent capable de remplacer l'atome d'oxygène par un atome de soufre pour obtenir le composé de formule ($I_B$) :

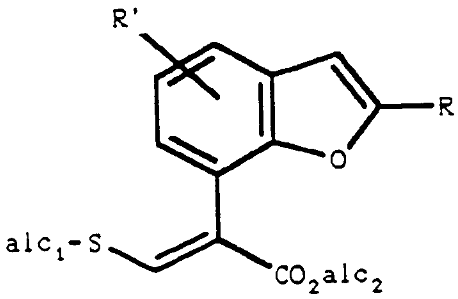

($I_B$)

- La réaction de formylation peut être réalisée en utilisant une base forte comme l'hydrure de sodium dans un solvant approprié suivi de l'action d'un formiate d'alkyle, de préférence méthyle, ou par action des acétals du diméthylformamide suivie d'une hydrolyse en milieu acide, ou encore par action d'une base forte comme le diisopropyle amidure de lithium suivie de l'action d'un halogénure de trialkylsilyle, l'éther d'énol silylé ainsi formé étant ensuite soumis à l'action d'un orthoformiate de trialkyle en présence de tétrachlorure de titane,
- la réaction d'alkylation a lieu en utilisant un alcool dans les conditions classiques ou un iodure d'alkyle sur l'énolate formé intermédiairement par action de l'hydrure de sodium sur le composé (III), ou encore un iodure d'alkyle en présence de carbonate de potassium dans l'acétone,
- la réaction d'échange oxygène-soufre est réalisée à l'aide d'un thiolate d'alkyle.

Les composés de formule (II) et (III) sont des produits nouveaux et sont en eux-mêmes un objet de la présente invention. Les composés de formule (II) et (III) préférés sont ceux décrits en exemple, notamment les composés de formule (II) et (III) décrits dans les exemples 1, 2, 11, 12, 14, 15, 16, 17, 18 et 19.

Les composés de formule (II) et (III) peuvent être préparés par les méthodes suivantes :

a)

```
                              SMe
                               |
A-Br --> A-CHO --> A-CH=C-S(O)-Me --> (II) --> (III)
(IV)     (V)          (VI)
```

b)

$A-CH_3$ --> $A-CH_2Br$ --> $A-CH_2CN$ --> $A-CH_2-COOH$ --> (II) --> (III)

(VII) (VIII) (IX) (X)

A représentant le groupe :

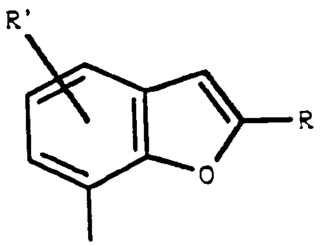

ou encore, lorsque R représente un radical thiazolyle éventuellement substitué par la méthode suivante :

c)

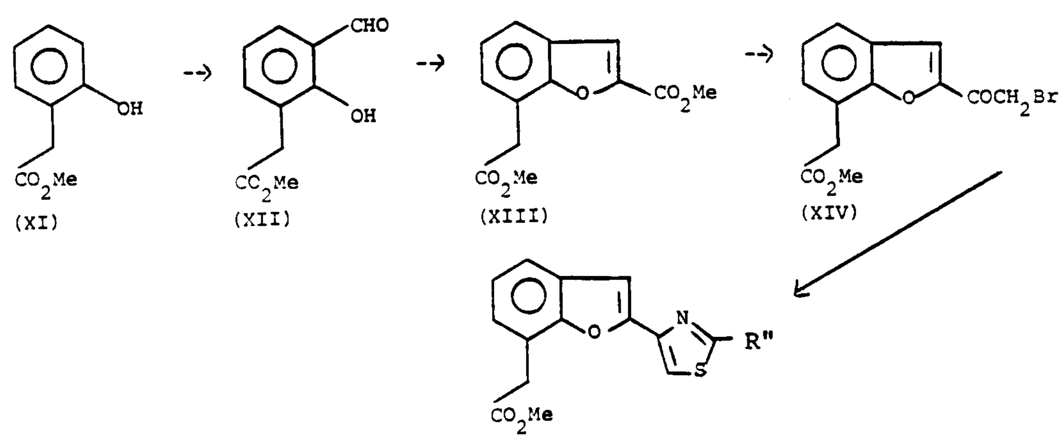

(XI) (XII) (XIII) (XIV)

(II).

Les composés de formule (IV), (V), (VII), (XII) et (XIV) sont obtenus par des méthodes décrites dans la littérature ou dans les préparations 1 à 4 et 14 à 19 décrites plus loin (voir C.E. CASTRO et Coll., Journal of Organic Chemistry (1966) 31 p. 4071-4078 ; MJ. COX et Coll. Tetrahedron (1975) 31 633 ; Pesticide Science (980) 11 p. 526-532 ; M. ROCHE et Coll. C.R. Acad. Scien. Paris C279 (1974) p. 663-666, Helvetica Acta (1974) 57 (5) p. 1381); Reimer et coll., Chem. Rev. 60 169-184 (1960) ; Corria et coll. Synthesis (1976) p. 194-195.

Les composés de l'invention présentent d'intéressantes propriétés pesticides qui permettent leur utilisation dans la lutte contre les parasites ; il peut s'agir par exemple de la lutte contre les parasites du sol, les parasites des végétaux, des locaux et les parasites des animaux à sang chaud.

L'invention a donc pour objet les compositions pesticides renfermant comme principe actif au moins un composé de formule (I) tel que défini précédemment, particulièrement les composés dont la préparation chimique est donnée en exemple et tout spécialement les composés des exemples 1, 2, 11, 12, 14, 15, 16, 17, 18, 19 et 28.

Les composés de formule (I) présentent d'intéressantes propriétés fongicides susceptibles d'être utilisées pour la protection vis-à-vis des champignons pathogènes. Il peut s'agir de la protection des plantes, de la protection des locaux ou des animaux.

Ces propriétés peuvent également être utilisées en hygiène et médecine humaine et animale.

Les composés de l'invention permettent de lutter contre de très nombreux champignons phytopathogènes, notamment contre : Erysiphe graminis, Sphaerotheca macularis, Sphaerotheca fuliginea, Podosphaera leucotricha, Uncinula necator, Helminthosporium spp., Rhynchosporium spp., Septoria spp., Pseudocercosporella herpotrichoides et Gaeumannomyces graminis, Ustilago spp., Cercospora arachidicola et Cercosporidium personatum, Cercospora species, Botrytis cinerea, Alternaria spp., Venturia inaequalis, Plasmopara viticola, Bremia lactucae, Peronospora spp., Pseudoperonospora humuli, Pseudoperonospora cubensis, Phytophthora spp. infestans, Phytophthora spp., Puccinia recondita, Thanatephorus cucumeris, Rhizoctonia spp. ou encore des champignons ou levures intéressant la santé humaine comme Candida albicans ou Trychophyton spp.

L'invention a donc pour objet les compositions fongicides renfermant comme principe actif au moins un composé de formule (I) tel que défini précédemment.

Les composés de l'invention présentent d'intéressantes propriétés insecticides, acaricides et nématicides.

Ils peuvent être utilisés notamment pour lutter contre les insectes dans le domaine agricole, pour lutter, par exemple, contre les pucerons, les larves de lépidoptères et les coléoptères.

Les composés de formule (I) peuvent également être utilisés pour lutter contre les insectes dans les locaux, pour lutter notamment contre les mouches, les moustiques et les blattes.

Les composés de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire.

Ces compositions peuvent se présenter sous forme de poudres, granulés, suspensions, émulsions, solutions, solutions pour aérosols, appâts ou autres préparations employés classiquement pour l'utilisation de ce genre de composés.

Outre le principe actif, ces compositions contiennent en général, un véhicule et/ou un agent tensioactif non ionique, assurant en outre une dispersion uniforme des substances constitutives du mélange. Le véhicule utilisé peut être un liquide tel que l'eau, l'alcool, les hydrocarbures ou autres solvants organiques, une huile minérale, animale ou végétale, une poudre telle que le talc, les argiles, les silicates, le kieselguhr.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables pour pulvérisation foliaire, contenant de 1 à 80 % de produit actif ou des liquides pour pulvérisation foliaire contenant de 1 à 500 g/l de principe actif. On peut également employer des poudres pour poudrage foliaire.

Pour l'usage nématicide, on utilise de préférence des liquides pour traitement des sols contenant de 300 à 500 g/l de principe actif.

Les exemples suivants illustrent l'invention sans toutefois la limiter.

**EXEMPLE 1 : alpha-[(E)-méthoxyméthylène] 2-(4-méthoxy phényl) 7-benzofuranneacétate de méthyle et isomère Z correspondant**

STADE A : alpha-[(E+Z)-hydroxyméthylène] 2-(4-méthoxy phényl) 7-benzofuranneacétate de méthyle

A une suspension de 1 g d'hydrure de sodium (à 50 % dans l'huile) dans 20 $cm^3$ de diméthylformamide, on introduit goutte à goutte à la température ambiante une solution renfermant 3 g de produit préparé ci-après (préparation 1), 14 $cm^3$ de formiate de méthyle et 35 $cm^3$ de diméthylformamide. On maintient sous agitation pendant 16 heures à la température ambiante. On verse sur une solution normale d'acide chlorhydrique, le pH du milieu doit être acide. On extrait à l'acétate d'éthyle. On sèche, concentre, et obtient le produit recherché, utilisé tel quel pour le stade suivant.

STADE B : alpha-[(E)-méthoxyméthylène] 2-(4-méthoxy phényl) 7-benzofuranneacétate de méthyle et isomère (Z) correspondant

On introduit 0,5 g d'hydrure de sodium à 50 % dans l'huile dans 20 $cm^3$ de diméthylformamide. On ajoute goutte à goutte une solution renfermant 12,3 g de alpha-[(E+Z)-hydroxyméthylène] 2-phényl 7-benzofuranneacétate de méthyle en solution dans 40 $cm^3$ de diméthylformamide. On maintient le mélange réactionnel sous agitation pendant 20 minutes. On ajoute en une seule fois 6,4 $cm^3$ d'iodure de méthyle. On maintient le mélange réactionnel sous agitation pendant une nuit à la température ambiante. On verse la solution obtenue dans l'eau ; on extrait à l'éther, sèche et amène à sec. On recueille 6,2 g de produit que l'on chromatographie sur silice en éluant avec le mélange hexane acétate d'éthyle (8-2). On obtient ainsi 2 g de produit isomère E fondant à 176 °C et 0,3 g de produit isomère Z fondant à 124 °C.

Préparation 1 : 2-(4-méthoxyphényl) 7-benzofuranneacétate de méthyle.

Stade A : (2-bromo phénoxy) 4′-méthoxy acétophénone

On ajoute 44,3 g de carbonate de potassium dans un mélange préparé à partir de 34 $cm^3$ de 2-bromo phénol, 150 $cm^3$ d'acétone et 75 g de 4′-méthoxy 2-bromo acétophénone dans 150 $cm^3$ d'acétone. On porte au reflux pendant 16 heures. On verse la solution obtenue dans l'eau. On extrait la phase aqueuse à l'acétate d'éthyle. On sèche, filtre et amène à sec. On obtient 101 g de produit recherché. F = 104 ° C.

Stade B : 7-bromo 2-(4-méthoxy phényl) benzofuranne.

On porte à 160 ° C pendant 1 heure, un mélange renfermant 101 g de produit préparé au stade A et 650 $cm^3$ d'acide polyphosphorique. On laisse revenir à la température ambiante, on verse sur la glace et extrait avec de l'acétate d'éthyle. On lave, décante, sèche et amène à sec. On obtient 82,8 g d'un produit que l'on purifie par chromatographie sur silice (éluant : hexane-éther isopropylique (98-2)). (Le produit, n'étant pas soluble dans l'éluant, est dissous dans l'acétate d'éthyle). On obtient ainsi 36,4 g de produit recherché.

Stade C : 2-(4-méthoxy phényl) 7-benzofurannecarbaldéhyde

On ajoute, à -70 ° C, 83 $cm^3$ de butyllithium dans un mélange de 36,4 g de produit préparé au stade précédent et 100 $cm^3$ de tétrahydrofuranne. On agite pendant 1 heure à -60 ° C et ajoute 24,2 $cm^3$ de N-formyl morpholine dans 50 $cm^3$ de tétrahydrofuranne. On maintient le mélange réactionnel sous agitation pendant 16 heures à -60 ° C. On ajoute du chlorure d'ammonium. On décante les deux phases obtenues. On extrait à l'acétate d'éthyle. On sèche et concentre. On obtient 35,12 g d'un produit que l'on purifie par chromatographie sur silice en éluant avec le mélange hexane-acétate d'éthyle (9-1). On obtient ainsi 13,8 g de produit recherché.

Stade D : 2-(4-méthoxy phényl) 7-benzofuranneacétate de méthyle.

On introduit 10,6 $cm^3$ de TRITON B® à 35 % dans le méthanol dans un mélange renfermant 15 g de produit préparé au stade C, 6 $cm^3$ de méthyl méthyl sulfinylméthyl sulfure et 25 $cm^3$ de tétrahydrofuranne. On porte la solution au reflux pendant 2 h 30. On verse dans l'eau et extrait à l'éther isopropylique, on sèche et concentre. On obtient 21,95 g d'un produit que l'on soumet à une hydrolyse méthanolique : on dissout ce produit dans 200 $cm^3$ d'une solution préparée par addition de 90,5 $cm^3$ de chlorure d'acétyle dans 366 $cm^3$ de méthanol refroidi à 0 ° C. Après 48 heures d'agitation à la température ambiante, on verse la solution obtenue dans l'eau, ajoute du carbonate acide de sodium. On extrait à l'éther, sèche et amène à sec. On obtient 16,5 g d'un produit que l'on chromatographie sur silice en éluant avec le mélange hexaneacétate d'éthyle (9-1). On obtient ainsi 11 g de produit recherché.

RMN : $CDCl_3$ 250 MHz ppm

| | |
|---|---|
| 2 $OCH_3$ | 3,73 ppm |
| | 3,87 |
| $-C_6H_3-\underline{CH_2}-CO$ | 4,00 ppm |
| $H_6$ | 6,89 |
| Aromatiques | 6,98 (2H) |
| | 7,78 (2H) |
| | 7,16 à 7,48 (3H) |

**EXEMPLE 2 : alpha-[(E)-méthoxyméthylène] 2-(3-méthoxy phényl) 7-benzofuranneacétate de méthyle et isomère Z correspondant**

En opérant comme à l'exemple 1, à partir du 2-(3-méthoxy phényl)] 7-benzofuranneacétate de méthyle, préparé ci-après, on a obtenu le produit E, F = 110 ° C et le produit Z, F = 130 ° C.

Préparation 2 : 2-(3-méthoxy phényl)] 7-benzofuranneacétate de méthyle.

Stade A : 2-(3-méthoxy phényl) 7-benzofurannecarbaldéhyde

On porte au reflux pendant 45 minutes, un mélange de 11 g de 2-hydroxy 3-iodo benzaldéhyde, 8,6 g de (3-méthoxy phényl) acétylène et 150 $cm^3$ de pyridine. On laisse revenir à la température ambiante. On ajoute de l'acétate d'éthyle. On lave à l'aide d'une solution acide, sèche et concentre. On recueille 16 g d'un produit que l'on chromatographie sur silice (éluant : hexane-éther isopropylique (7-3)) pour obtenir 9 g de produit recherché. F = 55-60 ° C.

Stade B : 2-(3-méthoxy phényl) 7-benzofuranneacétate de méthyle

On porte à ébullition pendant 1 heure un mélange renfermant 7,4 g de produit préparé au stade A, 4,4 g de méthyl méthylsulfinyl méthyl sulfure, 75 $cm^3$ de tétrahydrofuranne et 7 $cm^3$ de TRITON B®. On verse le mélange réactionnel dans l'eau, extrait au dichlorométhane, sèche et concentre. On obtient 10 g d'un produit que l'on dissout dans une solution chlorhydrique 2N dans le méthanol. On agite la solution obtenue pendant 24 heures. On traite avec du carbonate acide de sodium. On maintient sous agitation pendant une heure. On essore, extrait à l'éther, lave et concentre. On obtient 9 g d'un produit que l'on chromatographie sur silice (éluant : hexane-acétate d'éthyle (9-1)). On obtient 4,3 g de produit recherché utilisé tel quel.

| RMN : $CDCl_3$ 250 MHz ppm | |
|---|---|
| 2 $OCH_3$ | 3,72 (s) 3,87 (s) |
| $-C_6H_5-\underline{CH_2}-CO$ | 3,99 (s) |
| $H'_6$ | 6,88 (d,m) |
| $H_3$ | 7,01 (s) |
| Aromatiques | 7,17 à 7,5 (7H) |

**EXEMPLE 3 : alpha-[(E)-méthoxyméthylène] 2-phényl 7-benzofuranneacétate de méthyle et isomère Z correspondant**

En opérant comme à l'exemple 1, à partir de 2-phényl 7-benzofuranneacétate de méthyle, préparé ci-après préparation 3, on obtient le produit recherché isomère E, F = 126,4 ° C, isomère Z, F = 110,5 ° C.

Préparation 3 : 2-phényl 7-benzofuranneacétate de méthyle

Stade A : 2-phényl 7-benzofuranneacétonitrile

On ajoute 11 g de cyanure de potassium à 110 $cm^3$ de méthanol puis 23,5 g de 7-(bromométhyl) 2-phényl benzofuranne (Helvetica Chimica Acta Vol. 57, 5, 1974, p. 1381) dans 250 $cm^3$ de méthanol. On porte au reflux pendant 5 heures. On essore, filtre et concentre. On obtient des cristaux que l'on recristallise dans le flugène-1,1,3 au reflux. On obtient ainsi 5,78 g de produit recherché F = 65 ° C.

Stade B : Acide 2-phényl 7-benzofuranneacétique

On porte au reflux pendant 16 heures une suspension renfermant 24,68 g de produit préparé au stade A, 200 $cm^3$ de méthanol, 100 $cm^3$ d'eau et 160 $cm^3$ d'une solution de soude 10N. On élimine le méthanol. On reprend dans l'eau le produit obtenu et lave avec du chlorure de méthylène. On acidifie avec 150 $cm^3$ d'une solution d'acide chlorhydrique 12N. On essore le produit obtenu. On empâte à l'eau et sèche. On obtient 22 g du produit recherché fondant à 139,1 ° C.

Stade C : 2-phényl 7-benzofuranneacétate de méthyle

On porte au reflux pendant 1 heure une solution renfermant 20,45 g de produit préparé au stade précédent, 200 $cm^3$ de méthanol et 6 g d'acide para-toluènesulfonique. On amène à sec. On verse dans

l'eau. On extrait au chlorure de méthylène. On sèche, filtre, concentre. On obtient 21,33 g de produit recherché rf = 0,33 (éluant : hexane-chlorure de méthylène (60-40).

| RMN : $CDCl_3$ 250 MHz ppm | |
|---|---|
| $OCH_3$ | 3,72 (s) |
| $-C_6H_3-\underline{CH_2}-CO$ | 4,00 |
| $H'_6$ | 7,02 (s) |
| $H_3$ | 7,48 |
| Aromatiques | 7,2 à 7,85 (7H) |

**EXEMPLE 4 : alpha-[(E)-méthoxyméthylène] 2-4-méthoxy benzoyl) 7-benzofuranneacétate de méthyle et isomère Z correspondant**

En opérant comme aux exemples précédents à partir de 2-(4-méthoxy benzoyl) 7-benzofuranneacétate de méthyle, préparé ci-après, on obtient le produit recherché, rf = 0,07 (éluant : hexane-acétate d'éthyle (75-25)).

Préparation 4 : 2-(4-méthoxy benzoyl) 7-benzofuranneacétate de méthyle

Stade A : (4-méthoxy phényl) (7-méthyl 2-benzofurannyl) cétone

On introduit à 0°C, 99 $cm^3$ d'une solution normale de soude dans une solution de 13,45 g de 2-hydroxy 3-méthyl benzaldéhyde dans le méthanol. On agite à la température ambiante pendant 2 h 30. On amène à sec, reprend dans le méthanol, sèche et amène à sec. On empâte le produit dans un mélange chlorure de méthylène-éther isopropylique et acétate d'éthyle. On filtre et sèche. On obtient 15,4 g de phénate intermédiaire que l'on verse dans 60 $cm^3$ de dioxane préalablement chauffé à 95~100°C. On ajoute 20,53 g de 4'-méthoxy 2-bromo acétophénone en solution dans le dioxanne. On maintient à 95°C pendant 4 heures. On verse le milieu réactionnel sur une solution de tampon phosphate monopotassique. On extrait à l'acétate d'éthyle. On sèche et amène à sec. On purifie le produit obtenu par chromatographie sur silice en éluant avec le mélange hexane-chlorure de méthylène (50-50). On obtient 15,4 g de produit recherché. rf = 0,1.

Stade B : [7-(bromométhyl) benzofurannyl] (4-méthoxy phényl) cétone.

On ajoute 13,4 g de N-bromo succinimide et un peu d'azobisdiisobutyronitrile dans une solution renfermant 15,4 g de produit préparé au stade précédent dans 150 $cm^3$ de tétrachlorure de carbone. On porte au reflux pendant 18 heures à la lumière. On filtre et amène à sec le filtrat. On purifie les produits obtenus par chromatographie sur silice, en éluant avec le mélange hexane-chlorure de méthylène (50-50). On obtient le produit recherché. rf = 0,08.

Stade C : 2-(4-méthoxy benzoyl) 7-benzofuranneacétate de méthyle

On ajoute 3,8g de cyanure de potassium dans une solution renfermant 10 g de produit préparé au stade précédent dans 150 $cm^3$ de méthanol. On porte le mélange obtenu au reflux pendant 12 heures. On verse dans l'eau. On extrait au chlorure de méthylène. On lave à l'eau, sèche sur sulfate de magnésium et amène à sec. On obtient 8,48 g de produit que l'on verse dans 170 $cm^3$ de méthanol. On ajoute 50 $cm^3$ d'eau et 45 $cm^3$ d'une solution de soude 10 N. On porte au reflux pendant 4 heures. On évapore le méthanol. On verse dans l'eau et extrait au chlorure de méthylène. On acidifie la phase aqueuse avec de l'acide chlorhydrique concentré et extrait à l'acétate d'éthyle. On sèche et amène à sec. On obtient 7,37 g de produit que l'on verse dans 70 $cm^3$ de méthanol. On ajoute 700 mg d'acide p-toluènesulfonique. On porte au reflux pendant 1 h 15. On verse dans l'eau. On extrait au chlorure de méthylène. On lave à l'eau et amène à sec. On reprend le résidu avec 80 $cm^3$ d'acétone et 800 mg d'acide paratoluène sulfonique, agite 15 minutes, coule dans l'eau, extrait avec du chlorure de méthylène, sèche et évapore à sec. On obtient un produit que l'on purifie par chromatographie sur silice, en éluant avec le mélange hexane-acétate d'éthyle

(8-2). On obtient 6,65 g de produit recherché.

| | |
|---|---|
| <u>RMN</u> : $CDCl_3$ 250 MHz ppm | |
| 2 $OCH_3$ | 3,74 (s) |
| | 3,92 (s) |
| $-C_6H_3-\underline{CH_2}-CO$ | 4,03 (s) |
| H'$_6$ | 7,56 (s) |
| Aromatiques | 7,02 à 8,15 (7H) |

Ainsi les produits suivants ont été préparés en utilisant les modes opératoires détaillés déjà décrits.

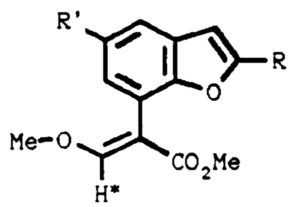

| Ex. | R | R' | E E/Z | F | RMN δH* ppm | Préparation |
|---|---|---|---|---|---|---|
| 1 | $4-CH_3O-C_6H_4$ | H | E | 176° | 7,72 | 1 |
| 1 | " | H | Z | 124° | 6,96 | 1 |
| 2 | $3-CH_3O-C_6H_4$ | H | E | 110° | 7,73 | 2 |
| 2 | " | H | Z | 130° | 6,97 | 2 |
| 3 | $C_6H_5-$ | H | E | 126° | 7,74 | 3 |
| 3 | " | H | Z | 110° | 6,96 | 3 |
| 4 | $4-CH_3O-C_6H_4CO-$ | H | E | huile | 7,74 | 4 |
| 4 | " | H | Z | huile | 7,04 | 4 |
| 5 | $C_6H_5CO-$ | H | E | 100-110° | 7,73 | 4 |
| 5 | " | H | Z | 140° | 7,05 | 4 |
| 6 | $2-CH_3O-C_6H_4-CO-$ | H | E | Résine | 7,69 | 4 |
| 6 | " | H | Z | Résine | 7,09 | 4 |
| 7 | $3-CH_3O-C_6H_4-CO-$ | H | E | 118° | 7,74 | 4 |
| 7 | " | H | Z | 84° | 7,07 | 4 |
| 8 | $4-Br\ 3-CF_3-C_6H_3-CO$ | H | E | 154° | 7,74 | 4 |
| 9 | $2-CH_3O-C_6H_4$ | H | E | 166° | 7,73 | 2 |
| 9 | " | H | Z | huile | 6,98 | 2 |
| 10 | $2-Cl-C_6H_4$ | H | E | 108° | 7,73 | 2 |
| 10 | " | H | Z | huile | 6,98 | 2 |
| 11 | $3-Cl-C_6H_4$ | H | E | huile | 7,75 | 2 |
| 11 | " | H | Z | 134° | 6,96 | 2 |
| 12 | $4-Cl-C_6H_4$ | H | E | 184° | 7,73 | 2 |
| 12 | " | H | Z | 132° | 6,94 | 2 |
| 13 | $C_6H_5-$ | I | E | 285° | 7,72 | 4 |
| 13 | " | I | Z | res. | 6,93 | 4 |

δH sont exprimés en ppm par rapport à une référence interne : le tétraméthylsilane. Toutes les solutions sont préparées dans $CDCl_3$.

**EXEMPLE 14 : alpha-[(E) méthoxyméthylène] 2-[4-bromo 3-(trifluorométhyl) phényl] benzofuranne acétate de méthylène et isomère Z correspondant**

On ajoute goutte à goutte à température ambiante, 2,59 g de 7-[2-[4-bromo 3-trifluorométhyl) phényl] benzofuranyl] acétate de méthyle préparé comme indiqué ci-dessous dans 600 mg d'hydrure de sodium en suspension dans 12 $cm^3$ de diméthylformamide. On ajoute 8,5 $cm^3$ de formiate de méthyle en solution dans 25 $cm^3$ de diméthylformamide, agite 1 heure. On ajoute goutte à goutte 3,9 $cm^3$ d'iodure de méthyle, agite 30 minutes, verse le milieu réactionnel dans une solution glacée de phosphate de sodium. On extrait à l'éther isopropylique, sèche, élimine le solvant sous pression réduite, chromatographie le résidu sur silice (éluant : hexane-acétate d'éthyle 85-15) et recueille 0,32 g d'isomère Z fondant à 114°C et 1,76 g d'isomère E que l'on empâte dans le pentane et obtient 1,63 g d'isomère E fondant à 134°C.

Spectre RMN ($CDCl_3$ 250 MHz ppm)

Isomère E 2-$OCH_3$ : 3,75 (s), 3,89 (s)
δH 7,75 (s)
Isomère Z 2-$OCH_3$ : 3,78 (s), 4,00 (s)
δH 6,97 (s)

**Préparation 14** : 7-[2-[4-bromo 3-trifluorométhyl) phényl] benzofuranyl] acétate de méthyle.

Stade A : 4-bromo 3-trifluorométhyl iodobenzène.

On ajoute 120 $cm^3$ d'acide chlorhydrique concentré à 60 g de 4-bromo 3-(trifluorométhyl) aniline en suspension dans 500 $cm^3$ d'eau. On chauffe à 80°C, refroidit à 0° ± 5°C et ajoute en 30 minutes 24,15 g de nitrite de sodium en solution dans 150 $cm^3$ d'eau. On agite pendant 1 heure, ajoute à cette solution en 40 minutes 127,4 g d'iodure de sodium dissous dans 130 $cm^3$ d'eau. On agite 16 heures à température ambiante, extrait le milieu réactionnel au chlorure de méthylène, lave la phase organique à l'aide d'une solution 3N de thiosulfate de sodium, puis à l'eau, sèche puis élimine les solvants sous pression réduite. Après purification par chromatographie sur silice (éluant : hexane-acétate d'éthyle 6-4), on obtient 83,95 g de produit attendu.

Stade B : [4-bromo 3-(trifluorométhyl) phényléthynyl] triméthylsilane.

Dans 30 g de produit obtenu au stade A en solution dans 90 $cm^3$ de diméthylformamide, on ajoute 16,7 $cm^3$ de triéthylamine, 11,83 $cm^3$ de triméthylsilylacétylène, 325 mg d'iodure de cuivre et 600 mg de bis-(triphénylphosphine) palladium dichlorure. On agite 1 heure, verse le milieu réactionnel dans l'eau glacée, extrait la phase aqueuse à l'éther isopropylique, sèche et élimine les solvants. Après chromatographie sur silice (éluant : hexane), on obtient 24,04 g de produit attendu.

Stade C : 4-bromo 3-(trifluorométhyl) phényl acétylène.

On ajoute à température ambiante 1 g de carbonate de potassium à 24 g du produit obtenu au stade B en solution dans 240 $cm^3$ de méthanol. On agite 45 minutes, évapore partiellement le méthanol, dilue au chlorure de méthylène et verse dans une solution aqueuse de bicarbonate de sodium à 10%. On extrait au chlorure de méthylène, sèche et élimine le solvant sous pression réduite. On obtient 17,73 g de produit attendu. F < 50°C.

Stade D : 7-[2-[4-bromo 3-(trifluorométhyl) phényl] benzofuranyl carboxaldéhyde.

Dans 2,3 g d'oxyde de cuivre (I) en suspension dans 70 $cm^3$ de pyridine, on ajoute 7 g du produit obtenu au stade C et 6,62 g de 3-iodo 2-hydroxy benzaldéhyde. On chauffe au reflux pendant 1 heure et demie, verse sur une solution d'acide chlorhydrique concentré, filtre, extrait au chlorure de méthylène, sèche et élimine le solvant sous pression réduite. On chromatographie le résidu sur silice (éluant : hexane-chlorure de méthylène 5-5) et obtient 6 g de produit que l'on empâte dans du pentane et recueille 4,85 g

de produit attendu.

Stade E : 7-[2-[4-bromo 3-trifluorométhyl) phényl] benzofuranyl] acétate de méthyle.

On chauffe au reflux pendant 5 heures et demie une solution comprenant 4,84 g de produit obtenu au stade D, 1,65 $cm^3$ de méthylsulfinylméthyl sulfure de méthyle et 3,5 $cm^3$ triton B* à 35% dans le méthanol. On verse sur une solution aqueuse saturée de chlorure de sodium, extrait à l'acétate d'éthyle, sèche et élimine les solvants. On obtient 7,22 g de 7-[2-[4-bromo 3-trifluorométhyl) phényl] benzofuranyl] 1-[-(méthylsulfinyl) méthylthio] éthylène intermédiaire que l'on dissout dans 60 $cm^3$ de méthanol, refroidit à 0°/+5°C et ajoute une solution méthanolique d'acide chlorhydrique 3,5N (préparé à partir de 21 $cm^3$ de chlorure d'acétyle dans 82 $cm^3$ de méthanol). On agite 4 heures à température ambiante, verse sur une solution aqueuse de bicarbonate de sodium à 10%, extrait à l'éther isopropylique, sèche, concentre à sec, chromatographie le résidu sur silice (éluant : hexane-chlorure de méthylène 5-5) et obtient après empâtage dans le pentane ou nouvelle chromatographie 2,65 g de produit attendu.

Spectre RMN ($CDCl_3$ 250 MHz ppm) :

$OCH_3$ 3,75 (s) ; $C_6H_3$-$CH_2$-CO 4,00 (s).

**EXEMPLE 15 : alpha-[(E)-(méthoxyméthylène) 2-[4-chloro (3-méthoxy) phényl] 7-benzofuranne acétate de méthylène et isomère Z correspondant.**

On opère comme à l'exemple 14 à partir de 3,8 g de 7-[2-[4-chloro (3-méthoxy)] phényl] benzofuranyl acétate de méthyle préparé comme indiqué ci-dessous, 1,1 g d'hydrure de sodium, 14,2 $cm^3$ de formiate de méthyle et 12,7 $cm^3$ d'iodure de méthyle. On obtient 5,8 g de produit brut que l'on chromatographie sur silice (éluant : hexane-chlorure de méthylène-acétone 90-05-05). On récupère 1,8 g d'isomère E fondant à 131,6°C et 0,7 g d'isomère Z fondant à 128,1°C.

Spectre RMN ($CDCl_3$ 250 MHz ppm) :

| | | | | |
|---|---|---|---|---|
| Isomère E | 3-$OCH_3$ | 3,74 (s) | 3,88 (s) | 3,98 (s) |
| | δH | 7,73 (s) | | |
| Isomère Z | 3-$OCH_3$ | 3,75 (s) | 3,98 (s) | 3,99 (s) |
| | δH | 6,94 (s) | | |

**Préparation 15** : 7-[2-[4-chloro (3-méthoxy)] phényl] benzofuranyl acétate de méthyle.

Stade A : [4-chloro 3-méthoxyphényléthynyl] triméthylsilane.

On opère comme au stade B de la préparation 14 à partir de 5 $cm^3$ de 4-chloro 3-méthoxy bromobenzène, 5,2 $cm^3$ de triméthylsilylacétylène et 260 mg de bis(triphénylphosphine) palladium dichlorure. On obtient 6,9 g de produit attendu.

Stade B : 4-chloro 3-méthoxyphénylacétylène.

On opère comme au stade C de la préparation 14 à partir de 8 g de produit obtenu au stade A ci-dessus et 0,46 g de carbonate de potassium. On récupère 5,2 g de produit attendu.

Stade C : 7-[2-[4-chloro (3-méthoxy) phényl] benzofuranyl formule.

On opère comme au stade D de la préparation 14 en utilisant au départ 5,2 g de produit obtenu au stade B ci-dessus, 7,3 g de 3-iodo 2-hydroxy benzaldéhyde en présence de 2,5 g d'oxyde de cuivre dans 75 $cm^3$ de pyridine. On obtient 9,8 g de produit attendu.

Stade D : 7-[2-[4-chloro (3-méthoxy)] phényl] benzofuranyl acétate de méthyle.

On opère comme au stade E de la préparation 14 au départ de 7,5 g de produit obtenu au stade C ci-dessus, 4 $cm^3$ de méthylsulfinylméthyle sulfure de méthyle et 4,7 $cm^3$ de Triton B* à 35% dans le méthanol. On obtient 8 g d'intermédiaire que l'on traite par une solution méthanolique d'acide chlorhydrique. On obtient après chromatographie sur silice (éluant : hexane-acétate d'éthyle 9-1) 4,7 g de produit attendu fondant à 97,7 °C.

Spectre RMN ($CDCl_3$ 250 MHz ppm) :

2-$OCH_3$ 4,00 (s,5H) 3,73 (s,3H).

**EXEMPLE 16 : alpha-[(E)-méthoxyméthylène] 7-[2-[4-(2-isopropylthiazolyl) benzofurannyl] acétate de méthyle.**

A 170 mg d'hydrure de sodium dans 2 $cm^3$ de diméthylformamide, on ajoute en 20 minutes 540 mg de 7-[2-[4-(2-isopropyl) thiazolyl] benzofurannyl] acétate de méthyle préparé comme indiqué ci-dessous dans 4 $cm^3$ de diméthylformamide et 1,7 $cm^3$ de formiate de méthyle. On abandonne 3 heures à température ambiante, ajoute 0,2 $cm^3$ d'iodure de méthyle, agite 1 heure, extrait à l'éther isopropylique, lave à l'eau, sèche, concentre, chromatographie le résidu sur silice (éluant : hexaneéther isopropylique 7-3 puis 8-2) et obtient 240 mg de produit attendu.

Spectre RMN ($CDCl_3$ ppm) :

| | | |
|---|---|---|
| 2-$OCH_3$-O | 3,72 (s) | 3,87 (s) |
| δH | 7,71 (s) | |

**Préparation 16** : 7-[2-[4-(2-isopropyl) thiazolyl] benzofurannyl] acétate de méthyle.

Stade A : 2-hydroxy 3-carbonyl phényl acétate de méthyle.

On chauffe à 120 °C, 24 g d'acide 2-hydroxy phényl acétique dans 50 $cm^3$ d'eau en présence de 50 g de soude. On ajoute en 3 heures, 100 $cm^3$ de chloroforme, refroidit et extrait avec 500 $cm^3$ de chloroforme et 500 $cm^3$ d'eau. On décante, élimine la solution chloroformique ; on acidifie la phase aqueuse à l'aide d'acide chlorhydrique concentré, extrait au chloroforme, concentre sous pression réduite et obtient 6 g d'aldéhyde intermédiaire auquel on ajoute 30 $cm^3$ de solution méthanolique d'acide chlorhydrique puis concentre sous pression réduite. On chromatographie le résidu sur silice (éluant : hexane-acétate d'éthyle 85-15) et obtient 2,9 g de produit attendu. F < 50 °C.

Stade B : 7-(2-acétylbenzofurannyl) acétate de méthyle.

On chauffe 15 minutes au reflux 5,5 g de produit obtenu au stade A dans 40 $cm^3$ de dioxanne avec 3,8 $cm^3$ de chloroacétone en présence de 5,9 g de carbonate de potassium. On filtre, concentre sous pression réduite et récupère après chromatographie du résidu sur silice (éluant : hexane-acétate d'éthyle 8-2) 4 g de produit attendu. F = 80 °C.

Stade C : 7-(2-bromoacétyl) benzofurannyl acétate de méthyle.

A 530 mg de produit préparé au stade B, on ajoute sous atmosphère inerte 2,97 $cm^3$ de solution molaire de 1,8-diazabicyclo[5.4.0.]undec-7-ène dans le chlorure de méthylène et de solution molaire de bromure de triméthylsilyle dans le chlorure de méthylène. On agite 6 heures au reflux, ajoute 0,5 $cm^3$ de chacune des 2 solutions, refroidit le milieu réactionnel à -60 °C et ajoute 2,26 $cm^3$ d'une solution molaire de brome dans le chlorure de méthylène. On agite 15 minutes à -60 °C, concentre sous pression réduite et chromatographie le résidu sur silice (éluant : acétate d'éthyle-hexane 3-7) et obtient 530 mg de produit attendu. F = 95 °C.

Stade D : 7-[2-[4-(2-isopropyl) thiazolyl] benzofurannyl] acétate de méthyle.

On chauffe au reflux pendant 45 minutes 850 mg de produit obtenu au stade C et 310 mg d'isopropyl thioamide dans 25 $cm^3$ de méthanol. On concentre sous pression réduite, reprend dans 30 $cm^3$ d'acétate d'éthyle et 10 $cm^3$ de solution aqueuse saturée en bicarbonate de sodium. On sèche, concentre de nouveau, chromatographie le résidu sur silice (éluant : hexane-acétate d'éthyle 9-1) et recueille 590 mg de produit attendu fondant à 60 ° C.

**EXEMPLE 17 : alpha-[(E)-méthoxyméthylène] 2-(3,4-méthylènedioxy) phényl 7-benzofuranne acétate de méthyle et isomère Z correspondant.**

On opère comme à l'exemple 1 au départ de 3,34 g de 7-[2-(3,4-méthylènedioxyphényl) benzofurannyl acétate de méthyle préparé comme indiqué ci-dessous, 1,05 g d'hydrure de sodium, 15 $cm^3$ de formiate de méthyle et 6,9 $cm^3$ d'iodométhane. On obtient 6,92 g de produit brut que l'on chromatographie sur silice (éluant : hexane-chlorure de méthylène-acétone 90-0,5-0,5). On obtient 1,66 g d'isomère E fondant à 154 ° C et 0,320 g d'isomère Z fondant à 189,7 ° C.

Spectre RMN ($CDCl_3$ 250 MHz ppm) :

| | | |
|---|---|---|
| Isomère E | $2-OCH_3$ | 3,73 (s) 3,87 (s) |
| | $\delta H$ | 7,72 (s) |
| Isomère Z | $2-OCH_3$ | 3,76 (s) 3,98 (s) |
| | $\delta H$ | 6,95 (s) |

**Préparation 17** : 7-[2-(3,4-méthylènedioxyphényl) benzofurannyl acétate de méthyle.

Stade A : 3,4-méthylènedioxy [1,1-dibromovinyl] benzène.

On ajoute 41,2 g de bromure de dibromo méthyl triphényl phosphonium puis 30 $cm^3$ de diméthylformamide à 8,98 g de terbutylate de potassium dans 90 $cm^3$ de tétrahydrofuranne. On agite la suspension pendant 1 heure à 20 ° C, refroidit à 0 ° C puis ajoute lentement 12 g de pipéronal dans 20 $cm^3$ de tétrahydrofuranne. On laisse revenir à température ambiante et maintient 3 heures sous agitation. On verse le milieu réactionnel sur une solution aqueuse d'hydrogénosulfate de sodium, extrait à l'acétate d'éthyle, sèche et concentre sous pression réduite. On obtient 15,97 g de produit attendu.

Stade B : 3,4-méthylènedioxy phényl acétylène.

On ajoute lentement à -65 ° C, 73 $cm^3$ de n-butyllithium dans l'hexane (1,6M) à 15,96 g de produit obtenu au stade A en solution dans 150 $cm^3$ de tétrahydrofuranne. On agite 2 heures à -65 ° C, verse sur une solution aqueuse saturée en hydrogénophosphate de sodium. On sépare par décantation la phase organique, sèche et concentre sous pression réduite. On obtient 7,6 g de produit attendu.

Stade C : 7-[2-(3,4-méthylènedioxy) phényl] benzofurannyl carboxaldéhyde.

On opère comme au stade D de la préparation 14 au départ de 5 g de produit obtenu au stade B précédent, 2,66 g d'oxyde de cuivre dans 60 $cm^3$ de pyridine et 8,02 g de 2-hydroxy 3-iodo benzaldéhyde. On obtient 3,55 g de produit attendu. F = 159,7 ° C.

Stade D : 7-[2-(3,4-méthylènedioxyphényl) benzofurannyl acétate de méthyle.

On opère comme au stade E de la préparation 14 au départ de 4,5 g de produit obtenu au stade C précédent, 3,1 g de Triton B*, 2,72 $cm^3$ de méthylsulfinylméthyl sulfure de méthyle. On obtient 7,5 g de 1-méthylsulfinyl 1-méthylthio 2-(3,4-méthylènedioxyphényl) benzofurannyl éthylène intermédiaire que l'on traite par une solution méthanolique d'acide chlorhydrique. On obtient 3,46 g de produit attendu.
F = 66,4 ° C.

Spectre RMN ($CDCl_3$ 250 MHz ppm) :

| | |
|---|---|
| $OCH_3$-O | 3,73 (s) |
| $C_6H_5$-$\underline{CH_2}$-CO | 3,98 (s) |

**EXEMPLE 18 : alpha-[(E)-méthoxyméthylène] 2-butyl 7-benzofuranne acétate de méthyle et isomère Z correspondant.**

On opère comme à l'exemple 14 au départ de 3,2 g de 7-[2-butyl) benzofurannyl acétate de méthyle préparé comme indiqué ci-dessous, 1,25 g d'hydrure de sodium, 17,6 $cm^3$ de formiate de méthyle et 8,2 $cm^3$ d'iodométhane. On obtient 7,56 g de produit brut que l'on chromatographie sur silice (éluant : hexane-chlorure de méthylène-acétone 90-0,5-0,5). On obtient 1,83 g d'isomère E et 0,350 g d'isomère Z fondant à 54,3 ° C.

Spectre RMN ($CDCl_3$ 250 MHz ppm) :

| | | | |
|---|---|---|---|
| Isomère E | 2-$OCH_3$ | 3,71 (s) | 3,85 (s) |
| | δH | 7,67 (s) | |
| Isomère Z | 2-$OCH_3$ | 3,74 (s) | 3,95 (s) |
| | δH | 6,91 (s) | |

**Préparation 18** : 7-[2-butyl) benzofurannyl acétate de méthyle.

Stade A : 7-(2-butyl) benzofurannyl carboxaldéhyde.

On opère comme au stade D de la préparation 14 au départ de 3,9 $cm^3$ de 1-hexyne, 2,66 g d'oxyde de cuivre (I) dans 60 $cm^3$ de pyridine et 8,02 g de 2-hydroxy 3-iodo benzaldéhyde. On obtient 5,08 g de produit attendu.

Stade B : 7-[2-butyl) benzofurannyl acétate de méthyle.

On opère comme au stade E de la préparation 14 au départ de 4,72 g de produit obtenu au stade A précédent, 4,1 g de Triton B* et 3,6 $cm^3$ de méthylsulfinylméthyl sulfure de méthyle. On obtient 7,0 g de 1-méthylsulfinyl 1-méthylthio 2-butyl benzofurannyléthylène intermédiaire que l'on traite par une solution méthanolique d'acide chlorhydrique. On obtient 5,29 g de produit brut que l'on chromatographie sur silice (éluant : hexane-chlorure de méthylène 8-2). On récupère 3,22 g de produit attendu.

Spectre RMN ($CDCl_3$ 250 MHz ppm) :

| | |
|---|---|
| $OCH_3$-O | 3,71 (s) |
| $C_6H_5$-$\underline{CH_2}$-CO | 3,92 (s) |

**EXEMPLE 19 : alpha-[(E)-méthoxyméthylène] 2-[2-[4-(trifluorométhoxy) phényl] 4-thiazolyl] 7-benzofuranne acétate de méthyle et isomère Z correspondant.**

On opère comme à l'exemple 14 en utilisant au départ 0,6 g de 7-[2-[4-(trifluorométhoxyphényl) thiazolyl] benzofurannyl] acétate de méthyle, 0,16 g d'hydrure de sodium, 3 $cm^3$ de formiate de méthyle, 1 $cm^3$ d'iodure de méthyle. On obtient 0,7 g de produit brut que l'on chromatographie sur silice (éluant :

heptane-acétate d'éthyle 8-2). On obtient 0,34 g d'isomère E et 0,12 g d'isomère Z. Après trituration dans l'hexane de l'isomère E, on obtient 0,27 g de produit fondant à 132 °C.

Spectre RMN ($CDCl_3$ 250 MHz ppm) :

| | | |
|---|---|---|
| Isomère E | 2-$OCH_3$ | 3,75 (s) 3,89 (s) |
| | δH | 7,74 (s) |

**Préparation 19** : 7-[2-[4-(trifluorométhoxyphényl) thiazolyl] benzofurannyl] acétate de méthyle.

On opère comme indiqué au stade D de la préparation 16 en utilsisant au départ du 7-(2-bromoacétyl) benzofurannyl acétate de méthyle et du trifluorométhoxythioamide.

En opérant comme indiqué dans les exemples précédents au départ des composés appropriés, on a préparé les produits des exemples 20 à 44.

**EXEMPLE 20 : (RS) 2-[(hydroxy (3-méthoxyphénylméthyl)] alpha-(méthoxyméthylène) 7-benzofuranne acétate de méthyle.**

**EXEMPLE 21 : (RS) 2-[hydroxy (4-méthoxyphénylméthyl)] alpha-(méthoxyméthylène) 7-benzofuranne acétate de méthyle.**

**EXEMPLE 22 : alpha-[(E)-méthoxyméthylène] 2-[[(E)-hydroxyimino] phénylméthyl)] 7-benzofuranne acétate de méthyle.**

**EXEMPLE 23 : alpha-[(E)-méthoxyméthylène] 2-[[(Z)-hydroxyimino] phénylméthyl)] 7-benzofuranne acétate de méthyle.**

**EXEMPLE 24 : alpha-[(E)-méthoxyméthylène] 2-(3-phénoxyphényl) 7-benzofuranne acétate de méthyle et isomère Z correspondant.**

**EXEMPLE 25 : alpha-[(E)-méthoxyméthylène] 2-(4-phénoxyphényl) 7-benzofuranne acétate de méthyle et isomère Z correspondant.**

**EXEMPLE 26 : alpha-[(E)-méthoxyméthylène] 2-[4-(2-phényl) thiazolyl] 7-benzofuranne acétate de méthyle et isomère Z correspondant.**

**EXEMPLE 27 : alpha-[(E)-méthoxyméthylène] 2-[2-chloro 4,5-méthylènedioxy] phényl] 7-benzofuranne acétate de méthyle et isomère Z correspondant.**

**EXEMPLE 28 : alpha-[(E)-méthoxyméthylène] 2-[4-(2-trifluorométhyl) thiazolyl] 7-benzofuranne acétate de méthyle.**

**EXEMPLE 29 : alpha-[(E)-méthoxyméthylène] 2-(4-biphénylyl) 7-benzofuranne acétate de méthyle.**

**EXEMPLE 30 : alpha-[(E)-méthoxyméthylène] 2-(3-biphénylyl) 7-benzofuranne acétate de méthyle.**

**EXEMPLE 31 : alpha-[(E)-méthoxyméthylène] 2-(1-naphtyl) 7-benzofuranne acétate de méthyle.**

**EXEMPLE 32 : alpha-[(E)-méthoxyméthylène] 2-(2-naphtyl) 7-benzofuranne acétate de méthyle.**

**EXEMPLE 33 : alpha-[(E)-méthylthiométhylène] 2-(4-méthoxyphényl) 7-benzofuranne acétate de méthyle.**

**EXEMPLE 34 : alpha-[(E)-méthoxyméthylène] 2-(4-nitrophényl) 7-benzofuranne acétate de méthyle et isomère Z correspondant.**

**EXEMPLE 35 : alpha-[(E)-méthoxyméthylène] 2-(2-méthylthio 4-thiazolyl) 7-benzofuranne acétate de méthyle et isomère Z correspondant.**

**EXEMPLE 36 : alpha-[(E)-méthoxyméthylène] 2-(2-diéthylamino 4-thiazolyl) 7-benzofuranne acétate de méthyle et isomère Z correspondant.**

**EXEMPLE 37 : alpha-[(E)-méthoxyméthylène] 2-trifluorométhyl 7-benzofuranne acétate de méthyle et isomère Z correspondant.**

**EXEMPLE 38 : alpha-[(E)-méthoxyméthylène] 2-heptafluoropropyl 7-benzofuranne acétate de méthyle et isomère Z correspondant.**

**EXEMPLE 39 : alpha-[(E)-méthoxyméthylène] 2-cyclohexyl 7-benzofuranne acétate de méthyle et isomère Z correspondant.**

**EXEMPLE 40 : alpha-[(E)-méthoxyméthylène] 2-isopropyl 7-benzofuranne acétate de méthyle et isomère Z correspondant.**

**EXEMPLE 41 : alpha-[(E)-méthoxyméthylène] 2-terbutyl 7-benzofuranne acétate de méthyle et isomère Z correspondant.**

**EXEMPLE 42 : alpha-[(E)-méthoxyméthylène] 2-sec-butyl 7-benzofuranne acétate de méthyle et isomère Z correspondant.**

**EXEMPLE 43 : alpha-[(E)-méthoxyméthylène] 2-(3,3-diméthyl 1-butynyl) 7-benzofuranne acétate de méthyle et isomère Z correspondant.**

**EXEMPLE 44 : alpha-[(E)-méthoxyméthylène] 2-(2-chloroéthényl) 7-benzofuranne acétate de méthyle et isomère Z correspondant.**

Ainsi les produits suivants ont été préparés en utilisant les modes opératoires détaillés décrits ci-dessus.

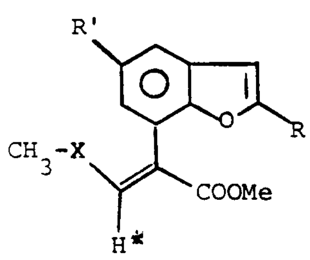

| Ex. | | R' | X | δ | F°C | RMN SH* ppm |
|---|---|---|---|---|---|---|
| 14 | 4-Br 3-$CF_3$-$C_6H_3$ | H | O | E | 134 | 7,75 |
| | | H | O | Z | 114 | 6,94 |
| 15 | 4-Cl 3-$CH_3O$-$C_6H_3$- | H | O | E | 131,6 | 7,73 |
| | | H | O | Z | 128,1 | 6,94 |
| 16 | 4-[2-$(CH_3)_2CH$-$C_3HNS$]- | H | O | E | huile | 7,71 |
| 17 | 3,4-(-O-$CH_2$-O)$C_6H_3$- | H | O | E | 154 | 7,72 |
| | | H | O | Z | 189,7 | 6,95 |
| 18 | n-$C_4H_9$- | H | O | E | huile | 7,67 |
| | | H | O | Z | 54,3 | 6,91 |
| 19 | 4-[2-[4-$CF_3O$-$C_6H_4$]$C_3HNS$- | H | O | E | 132 | 7,74 |
| 20 | 3-$CH_3O$-$C_6H_4$-CHOH- | H | O | E | résine | 7,62 |
| 21 | 4-$CH_3O$-$C_6H_4$-CHOH- | H | O | E | huile | 7,63 |
| 22 | $C_6H_5$-C=NOH- (E) | H | O | E | résine | 7,68 |
| 23 | $C_6H_5$-C=NOH- (Z) | H | O | E | 124 | 7,64 |
| 24 | 3-$C_6H_5$-$OC_6H_4$- | H | O | E | 132,4 | 7,69 |
| | | H | O | Z | résine | 7,00 |
| 25 | 4-$C_6H_5$-$OC_6H_4$- | H | O | E | 149,9 | 7,72 |
| | | H | O | Z | 152,8 | 6,96 |
| 26 | 4-(2-$C_6H_5$-$C_3HNS$)- | H | O | E | 165 | 7,59 |
| | | H | O | Z | huile | 6,95 |
| 27 | 2-Cl 3,4(-$OCH_2$-O)$C_6H_3$- | H | O | E | 150,5 | 7,73 |
| | | H | O | Z | 237,3 | 6,96 |
| 28 | 4-[2-$CF_3$-$C_3HNS$]- | H | O | E | 95 | 7,73 |
| 29 | 4-$C_6H_5$-$C_6H_4$- | H | O | E | 223,3 | 7,75 |
| 30 | 3-$C_6H_5$-$C_6H_4$- | H | O | E | 102 | 7,74 |
| 31 | 1-$C_{10}H_7$ | H | O | E | 132,2 | 7,73 |
| 32 | 2-$C_{10}H_7$ | H | O | E | | |

| Ex. | | R' | X | δ | F°C | RMN SH* ppm |
|---|---|---|---|---|---|---|
| 33 | 4-CH3O-$C_6H_4$ | H | S | E | 134-138 | 8,02 |
| | | | | | | - |
| 34 | 4-NO2-$C_6H_4$- | H | O | E | - | 7,75 |
| | | | | Z | - | - |
| 35 | 4-[2-($CH_3S$-$C_3HNS$]- | H | O | E | - | 7,72 |
| | | | | Z | - | - |
| 36 | 4-[2-$(C_2H_5)_2N$-$C_3HNS$]- | H | O | E | - | 7,74 |
| | | | | Z | - | - |
| 37 | -$CF_3O$- | H | O | E | huile | 7,81 |
| | | | | Z | - | - |
| 38 | -$CF_2$-$CF_2$-$CF_3$ | H | O | E | huile | 7,82 |
| | | | | Z | - | - |
| 39 | -$C_6H_{11}$ | H | O | E | résine | 7,70 |
| | | | | Z | - | - |
| 40 | -CH-$(CH_3)_2$ | H | O | E | huile | 7,71 |
| | | | | Z | - | - |
| 41 | -C-$(CH_3)_3$ | H | O | E | résine | 7,74 |
| | | | | Z | - | - |
| 42 | -$(CH_3)$CH-$C_2H_5$ | H | O | E | huile | 7,73 |
| | | | | Z | - | - |
| 43 | -C≡C-$(CH_3)_3$ | H | O | E | huile | 7,69 |
| | | | | Z | - | - |
| 44 | -C=CH¯¯Cl | H | O | E | résine | 7,81 |
| | | | | Z | - | - |

**EXEMPLE 45 : Préparation d'un concentré soluble.**

On a préparé un concentré soluble renfermant comme principe actif le produit de l'exemple 1 (E).

**EXEMPLE 46 : Préparation d'un concentré émulsifiable.**

On a préparé un concentré émulsifiable renfermant comme principe actif le produit de l'exemple 11 (E).

## ACTIVITE BIOLOGIOUE :

I - Activité fongicide

**Essais sur Plasmopara viticola**

Les jeunes plants de vigne issus de boutures (variété Grenache N, clone 70) sont cultivés en serre (température de jour : 30 °C, température de nuit : 25 °C) sur mélange terre/terreau/sable, (1/3-1/3-1/3). Deux jours avant l'essai, les plants sont transportés en chambre de culture (mêmes conditions de température, humidité : 60 % le jour, 80 % la nuit). Le produit est dissous dans la "matrice A" à une concentration de 100 ppm juste avant l'utilisation. Le traitement se fait par pulvérisation de la solution sur les feuilles jusqu'à rétention maximale. La contamination est faite avec une suspension de zoosporanges de Plasmopara viticola prélevés immédiatement avant l'essai (50.000 zoosporanges par ml). Les gouttes de suspension (20 microlitres) sont déposées à la face abaxiale des feuilles. Les plants sont ensuite maintenus pendant 24 heures dans une atmosphère saturée en humidité, puis ramenés à l'humidité de la chambre de culture (60 % le jour, 80 % la nuit). La lecture s'effectue dix jours après la contamination, par mesure du développement des îlots de sporangiothiores à la surface abaxiale des feuilles. L'efficacité du produit est calculée par rapport à un témoin non traité. Le même type de test est réalisé sur cépage Chardonnay.

Les résultats obtenus en % d'efficacité sur plasmopara viticola à 100 ppm de matière active sont les suivants :

| Produit | Cépage grenache | Cépage chardonnay |
|---|---|---|
| Ex. n° 1 (E) | 90 | n. d. |
| Ex. n° 1 (Z) | 100 | n. d. |
| Ex. n° 2 (E) | 100 | 100 |
| Ex. n° 10 (E) | 100 | 90 |
| Ex. n° 11 (E) | 100 | 100 |
| Ex. n° 12 (E) | 100 | 100 |

n. d. = non déterminé

Exemple de composition de "MATRICE A" :

| | |
|---|---|
| Solvesso 150 | 70,0 g |
| NAPSOL PM1 | 850,0 g |
| SURFAROX HRH 40 c | 52,0 g |
| ECD 1604 | 28,0 g |
| | 1000,0 g |

II - Activité insecticide :

a) **Etude de l'effet sur larves de SPODOPTERA LITTORALIS (S.L.) par contact et ingestion.**

On utilise des larves du stade L3 de SPODOPTERA LITTORALIS. On opère à 22 °C dans des conditions d'humidité relative de 50 %. On utilise des boîtes de PETRI renfermant un rond de papier filtre

humidifié, dans chaque boîte on place deux feuilles de haricot traitées par une solution hydroacétonique (50-50) renfermant le produit à tester.

On fait le comptage des larves mortes au bout de 7 jours. On recherche la concentration $CL_{50}$ ; les résultats sont exprimés en ppm.

**b) Etude de l'effet létal sur Musca Domestica (M.D.) :**

On utilise des mouches femelles âgées de 6 jours pesant de 18 à 20 mg.

On opère à 22°C dans des conditions d'humidité relative de 50%.

Les produits sont administrés par application topique de solution acétonique à 1 g/l sur le thorax dorsal des insectes.

On recherche la dose $CL_{50}$. Les résultats sont exprimés en ppm.

**c) Etude de l'activité sur Aphis cracivora :**

On pulvérise 2 $cm^3$ de solution hydro-acétonique (50/50) sur des feuilles de fève (vicia fabae) jusqu'à ruissellement. Après séchage, on dépose 15 femelles aptères d'Aphis cracivora. L'ensemble est maintenu sur du papier filtre humide à l'intérieur d'une boîte de Pétri. 24 heures après le début du contact, on procède à la détermination du nombre d'insectes morts.

On recherche la dose $CL_{50}$. Les résultats sont exprimés en ppm.

III - Etude acaricide des composés de l'invention.

On utilise des plants de haricot comportant 2 feuilles infestées de 30 femelles de Tétranychus Urticae par feuille et mis sous bonette aérée sous plafond lumineux en lumière constante. Les plants sont traités au pistolet Fisher : 4 ml de solution toxique par plant d'un mélange à volume égal d'eau et d'acétone. On laisse sécher pendant 1/2 heure puis on procède à l'infestation. Les contrôles de mortalité sont effectués au bout de 3 jours.

On recherche la dose $CL_{50}$.

Les résultats sont regroupés dans le tableau ci-dessous, en tenant compte des valeurs suivantes :

A : $CL_{50} < 250$ ppm

B : $250 < CL_{50} \leqq 1000$ ppm

C : $CL_{50} > 1000$ ppm

| Produit de l'exemple | Mouche | Spodoptera | Aphis | Tetranychus |
|---|---|---|---|---|
| 3 (E) | - | B | - | - |
| 3 (Z) | - | B | - | - |
| 1 (E) | - | B | - | A |
| 12 (E) | A | - | - | A |
| 11 (E) | - | B | - | - |
| 1 (Z) | - | B | - | A |
| 14 (E) | A | B | - | A |
| 24 (E) | A | - | - | - |
| 25 (E) | A | - | - | - |
| 17 (E) | A | A | - | - |
| 15 (E) | A | B | A | B |
| 19 (E) | - | A | - | - |
| 18 (E) | - | - | A | A |
| 16 (E) | - | - | - | A |

**Revendications**

**Revendications pour les Etats contractants suivants : CH, DE, FR, GB, IT, LI, NL**

**1.** Les composés de formule (I) :

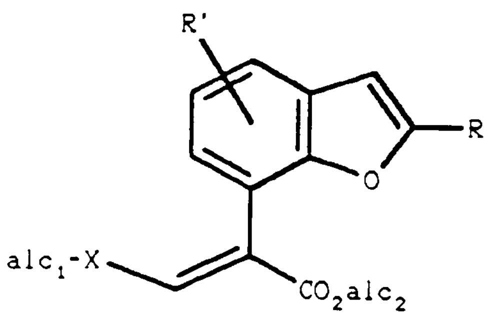

(I)

dans laquelle :

- R représente un atome d'hydrogène, un radical alkyle, alcényle ou alcynyle renfermant jusqu'à 8 atomes de carbone éventuellement substitué, un radical cycloalkyle ou cycloalcényle renfermant jusqu'à 8 atomes de carbone éventuellement substitué, un radical aryle renfermant jusqu'à 18 atomes de carbone éventuellement substitué, un radical arylalkyle renfermant jusqu'à 24 atomes de carbone éventuellement substitué, un radical benzoyle éventuellement substitué ou un radical hétérocyclique éventuellement substitué,
- $alc_1$ et $alc_2$ identiques ou différents représentent un radical alkyle renfermant jusqu'à 8 atomes de carbone éventuellement substitué,
- X représente un atome d'oxygène ou de soufre,
- R′ en position quelconque sur le noyau phényle représente un atome d'hydrogène ou un atome d'halogène,

  la géométrie de la double liaison étant E ou Z ou un mélange E et Z.

2. Les composés de formule (I) tels que définis à la revendication 1 dans lesquels X représente un atome d'oxygène.

3. Les composés de formule (I) tels que définis à la revendication 1 ou 2 dans lesquels R′ représente un atome d'hydrogène.

4. Les composés de formule (I) tels que définis à l'une quelconque des revendications 1 à 3 dans lesquels $alc_1$ et $alc_2$ représentent chacun un radical méthyle.

5. Les composés de formule (I) tels que définis à l'une quelconque des revendications 1 à 4 dans lesquels R représente un radical alkyle renfermant jusqu'à 8 atomes de carbone.

6. Les composés de formule (I) tels que définis à la revendication 5 dans lesquels R représente un radical n-butyle.

7. Les composés de formule (I) tels que définis à l'une quelconque des revendications 1 à 4 dans lesquels R représente un radical phényle éventuellement substitué par un ou plusieurs radicaux choisis dans le groupe constitué par les atomes d'halogène, les radicaux phénoxy, thiazolyle et alkyloxy renfermant jusqu'à 6 atomes de carbone et alkyle substitué par un ou plusieurs atomes d'halogène ou R représente un radical phényle substitué sur deux carbones adjacents par un radical alkylènedioxy.

8. Les composés de formule (I) tels que définis à la revendication 7 dans lesquels R représente un radical phényle substitué par un radical alkyloxy renfermant jusqu'à 4 atomes de carbone, par un atome d'halogène ou simultanément par ces deux substituants.

9. Les composés de formule (I) tels que définis à la revendication 8 dans lesquels R représente un radical phényle substitué par un radical méthoxy.

10. Les composés de formule (I) tels que définis à la revendication 8 dans lesquels R représente un radical phényle substitué par un atome de chlore.

11. Les composés de formule (I) tels que définis à la revendication 8 dans lesquels R représente un radical phényle substitué par un atome de brome.

12. Les composés de formule (I) tels que définis à la revendication 7 dans lesquels R représente un radical phényle substitué par un radical trifluorométhyle.

13. Les composés de formule (I) tels que définis à la revendication 7 dans lesquels R représente un radical 3,4-méthylènedioxyphényle.

14. Les composés de formule (I) tels que définis à l'une quelconque des revendications 1 à 4 dans lesquels R représente un radical thiazolyle éventuellement substitué par un radical alkyle linéaire ou ramifié renfermant jusqu'à 8 atomes de carbone ou par un radical phényle éventuellement substitué.

15. Les composés de formule (I) tels que définis à la revendication 14 dans lesquels R représente un radical thiazolyle substitué par un radical isopropyle.

16. Les composés de formule (I) tels que définis à la revendication 14 dans lesquels R représente un radical thiazolyle substitué par un radical trifluorométhoxyphényle.

17. Les composés de formule (I) tels que définis à l'une quelconque des revendications 1 à 16 dans lesquels la double liaison éther d'énol est de géométrie E.

18. Les composés de formule (I) tels que définis à la revendication 17 dont les noms suivent :
   - alpha-[(E)-méthoxyméthylène] 2-(4-méthoxy phényl) 7-benzofuranneacétate de méthyle
   - alpha-[(E)-méthoxyméthylène] 2-(3-méthoxy phényl) 7-benzofuranneacétate de méthyle
   - alpha-[(E)-méthoxyméthylène] 2-(3-chloro phényl) 7-benzofuranneacétate de méthyle
   - alpha-[(E)-méthoxyméthylène] 2-(2-chloro phényl) 7-benzofuranneacétate de méthyle,

- alpha-[(E) méthoxyméthylène] 2-[4-bromo 3-(trifluorométhyl) phényl] benzofuranne acétate de méthylène
- alpha-[(E)-(méthoxyméthylène] 2-[4-chloro (3-méthoxy) phényl] 7-benzofuranne acétate de méthylène
- alpha-[(E)-méthoxyméthylène] 7-[2-[4-(2-isopropylthiazolyl) benzofurannyl] acétate de méthyle
- alpha-[(E)-méthoxyméthylène] 2-(3,4-méthylènedioxy) phényl 7-benzofuranne acétate de méthyle
- alpha-[(E)-méthoxyméthylène] 2-butyl 7-benzofuranne acétate de méthyle
- alpha-[(E)-méthoxyméthylène] 2-[2-[4-(trifluorométhoxy) phényl] 4-thiazolyl] 7-benzofuranne acétate de méthyle,
- alpha-[(E)-méthoxyméthylène] 2-[4-(2-trifluorométhyl) thiazolyl] 7-benzofuranne acétate de méthyle.

**19.** Procédé de préparation des composés de formule (I) tels que définis à l'une quelconque des revendications 1 à 18, caractérisé en ce que l'on soumet un composé de formule (II) :

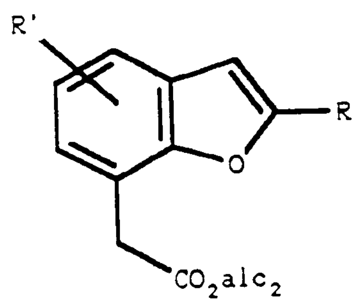

(II)

dans laquelle R, R' et $alc_2$ conservent leur signification indiquée à la revendication 1, à l'action d'un agent de formylation pour obtenir le composé de formule (III) :

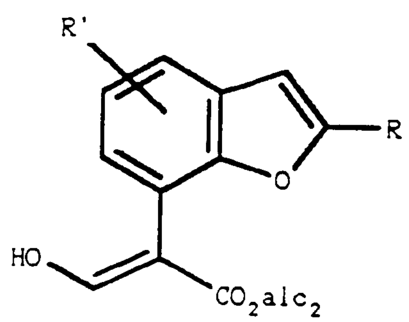

(III)

que l'on soumet à l'action d'un agent d'alkylation pour obtenir le composé de formule ($I_A$) :

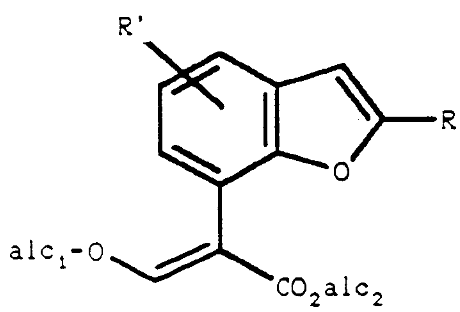

($I_A$)

dans laquelle $alc_1$ conserve la signification indiquée à la revendication 1, que l'on soumet si désiré à l'action d'un agent capable de remplacer l'atome d'oxygène par un atome de soufre pour obtenir le composé de formule ($I_B$) :

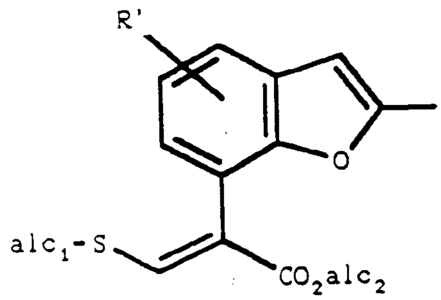

$(I_B)$

20. Les composés de formule (II) et (III) définis à la revendication 19, à titre de produits industriels nouveaux.

21. Les compositions pesticides renfermant comme principe actif au moins un composé de formule (I) tel que défini à l'une quelconque des revendications 1 à 18.

22. Les compositions insecticides renfermant comme principe actif au moins un composé de formule (I) tel que défini à l'une quelconque des revendications 1 à 18.

23. Les compositions acaricides renfermant comme principe actif au moins un composé de formule (I) tel que défini à l'une quelconque des revendications 1 à 18.

24. Les compositions fongicides renfermant comme principe actif au moins un composé de formule (I) tel que défini à l'une quelconque des revendications 1 à 18.

**Revendications pour l'Etat contractant suivant : ES**

1. Procédé pour préparer les composés de formule (I) :

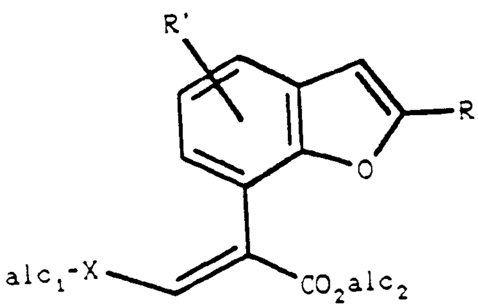

(I)

dans laquelle :

- R représente un atome d'hydrogène, un radical alkyle, alcényle ou alcynyle renfermant jusqu'à 8 atomes de carbone éventuellement substitué, un radical cycloalkyle ou cycloalcényle renfermant jusqu'à 8 atomes de carbone éventuellement substitué, un radical aryle renfermant jusqu'à 18 atomes de carbone éventuellement substitué, un radical arylalkyle renfermant jusqu'à 24 atomes de carbone éventuellement substitué, un radical benzoyle éventuellement substitué ou un radical hétérocyclique éventuellement substitué,
- $alc_1$ et $alc_2$ identiques ou différents représentent un radical alkyle renfermant jusqu'à 8 atomes de carbone éventuellement substitué,
- X représente un atome d'oxygène ou de soufre,
- R′ en position quelconque sur le noyau phényle représente un atome d'hydrogène ou un atome d'halogène, la géométrie de la double liaison étant E ou Z ou un mélange E et Z, caractérisé en ce que l'on soumet un composé de formule (II) :

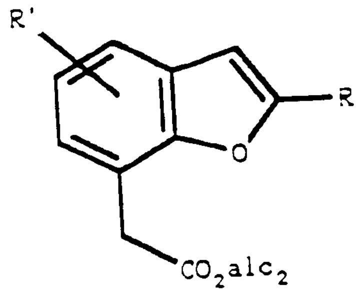

(II)

dans laquelle R, R' et $alc_2$ conservent leur signification indiquée précédemment, à l'action d'un agent de formylation pour obtenir le composé de formule (III) :

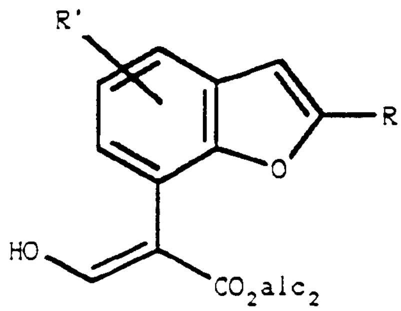

(III)

que l'on soumet à l'action d'un agent d'alkylation pour obtenir le composé de formule ($I_A$) :

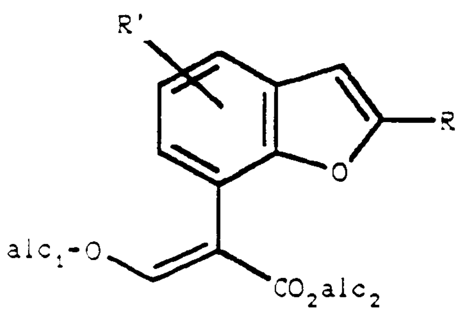

($I_A$)

dans laquelle $alc_1$ conserve la signification indiquée précédemment, que l'on soumet si désiré à l'action d'un agent capable de remplacer l'atome d'oxygène par un atome de soufre pour obtenir le composé de formule ($I_B$) :

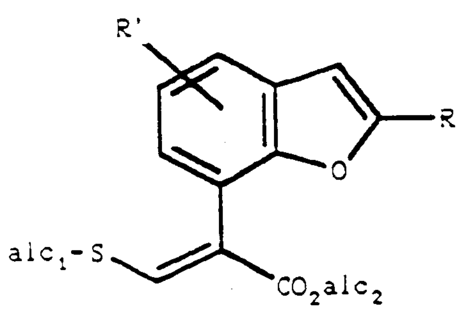

($I_B$)

2. Procédé selon la revendication 1, caractérisé en ce que l'on utilise au départ un produit de formule (II) dans laquelle R′ représente un atome d'hydrogène.

3. Procédé selon la revendication 1 ou 2, caractérisé en ce que l'on utilise au départ un produit de formule (II) dans laquelle $alc_2$ représente un radical méthyle et que l'on fait réagir un agent de méthylation sur le produit de formule (III) obtenu.

4. Procédé selon l'une quelconque des revendications 1 à 3, caractérisé en ce que l'on utilise au départ un produit de formule (II) dans laquelle R représente un radical alkyle renfermant jusqu'à 8 atomes de carbone.

5. Procédé selon la revendication 4, caractérisé en ce que l'on utilise au départ un produit de formule (II) dans laquelle R représente un radical n-butyle.

6. Procédé selon l'une quelconque des revendications 1 à 3, caractérisé en ce que l'on utilise au départ un produit de formule (II) dans laquelle R représente un radical phényle éventuellement substitué par un ou plusieurs radicaux choisis dans le groupe constitué par les atomes d'halogène, les radicaux phénoxy, thiazolyle et alkyloxy renfermant jusqu'à 6 atomes de carbone et alkyle substitué par un ou plusieurs atomes d'halogène ou R représente un radical phényle substitué sur deux carbones adjacents par un radical alkylènedioxy.

7. Procédé selon la revendication 6, caractérisé en ce que l'on utilise au départ un produit de formule (II) dans laquelle R représente un radical phényle substitué par un radical alkyloxy renfermant jusqu'à 4 atomes de carbone, par un atome d'halogène ou simultanément par ces deux substituants.

8. Procédé selon la revendication 7, caractérisé en ce que l'on utilise au départ un produit de formule (II) dans laquelle R représente un radical phényle substitué par un radical méthoxy.

9. Procédé selon la revendication 6, caractérisé en ce que l'on utilise au départ un produit de formule (II) dans laquelle R représente un radical phényle substitué par un atome de chlore.

10. Procédé selon la revendication 6, caractérisé en ce que l'on utilise au départ un produit de formule (II) dans laquelle R représente un radical phényle substitué par un atome de brome.

11. Procédé selon la revendication 6, caractérisé en ce que l'on utilise au départ un produit de formule (II) dans laquelle R représente un radical phényle substitué par un radical trifluorométhyle.

12. Procédé selon la revendication 6, caractérisé en ce que l'on utilise au départ un produit de formule (II) dans laquelle R représente un radical 3,4-méthylènedioxyphényle.

13. Procédé selon l'une quelconque des revendications 1 à 3, caractérisé en ce que l'on utilise au départ un produit de formule (II) dans laquelle R représente un radical thiazolyle éventuellement substitué par un radical alkyle linéaire ou ramifié renfermant jusqu'à 8 atomes de carbone ou par un radical phényle éventuellement substitué.

14. Procédé selon la revendication 12, caractérisé en ce que l'on utilise au départ un produit de formule (II) dans laquelle R représente un radical thiazolyle substitué par un radical isopropyle.

15. Procédé selon la revendication 12, caractérisé en ce que l'on utilise au départ un produit de formule (II) dans laquelle R représente un radical thiazolyle substitué par un radical trifluorométhoxyphényle.

16. Procédé selon l'une quelconque des revendications 1 à 15, caractérisé en ce que l'on choisit le produit de formule (II) et les réactifs de manière telle que l'on prépare l'un des produits dont les noms suivent :
    - alpha-[(E)-méthoxyméthylène] 2-(4-méthoxy phényl) 7-benzofuranneacétate de méthyle
    - alpha-[(E)-méthoxyméthylène] 2-(3-méthoxy phényl) 7-benzofuranneacétate de méthyle
    - alpha-[(E)-méthoxyméthylène] 2-(3-chloro phényl) 7-benzofuranneacétate de méthyle
    - alpha-[(E)-méthoxyméthylène] 2-(2-chloro phényl) 7-benzofuranneacétate de méthyle,

- alpha-[(E) méthoxyméthylène] 2-[4-bromo 3-(trifluorométhyl) phényl] benzofuranne acétate de méthylène
- alpha-[(E)-(méthoxyméthylène) 2-[4-chloro (3-méthoxy) phényl] 7-benzofuranne acétate de méthylène
- alpha-[(E)-méthoxyméthylène] 7-[2-[4-(2-isopropylthiazolyl) benzofurannyl] acétate de méthyle
- alpha-[(E)-méthoxyméthylène] 2-(3,4-méthylènedioxy) phényl 7-benzofuranne acétate de méthyle
- alpha-[(E)-méthoxyméthylène] 2-butyl 7-benzofuranne acétate de méthyle
- alpha-[(E)-méthoxyméthylène] 2-[2-[4-(trifluorométhoxy) phényl] 4-thiazolyl] 7-benzofuranne acétate de méthyle.

**Claims**

**Claims for the following Contracting States : CH, DE, FR, GB, IT, LI, NL**

1. The compounds of formula (I):

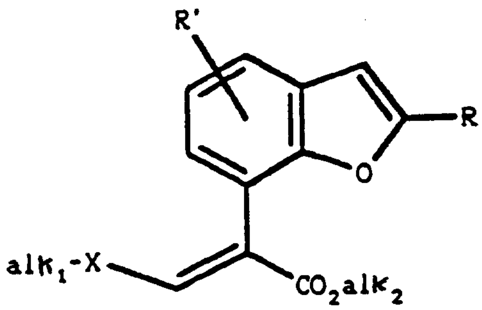

(I)

in which:

- R represents a hydrogen atom, an optionally substituted alkyl, alkenyl or alkynyl radical containing up to 8 carbon atoms, an optionally substituted cycloalkyl or cycloalkenyl radical containing up to 8 carbon atoms, an optionally substituted aryl radical containing up to 18 carbon atoms, an optionally substituted arylalkyl radical containing up to 24 carbon atoms, an optionally substituted benzoyl radical or an optionally substituted heterocyclic radical,
- $alk_1$ and $alk_2$, identical or different, represent an optionally substituted alkyl radical containing up to 8 carbon atoms,
- X represents an oxygen or sulphur atom,
- R' in any position on the phenyl nucleus represents a hydrogen atom or a halogen atom,
the geometry of the double bond being E or Z or an E and Z mixture.

2. The compounds of formula (I) as defined in claim 1 in which X represents an oxygen atom.

3. The compounds of formula (I) as defined in claim 1 or 2 in which R' represents a hydrogen atom.

4. The compounds of formula (I) as defined in any one of claims 1 to 3 in which $alk_1$ and $alk_2$ each represent a methyl radical.

5. The compounds of formula (I) as defined in any one of claims 1 to 4 in which R represents an alkyl radical containing up to 8 carbon atoms.

6. The compounds of formula (I) as defined in claim 5 in which R represents an n-butyl radical.

7. The compounds of formula (I) as defined in any one of claims 1 to 4 in which R represents a phenyl radical optionally substituted by one or more radicals chosen from the group constituted by halogen atoms, phenoxy, thiazolyl and alkyloxy radicals containing up to 6 carbon atoms and alkyl radicals substituted by one or more halogen atoms or R represents a phenyl radical substituted on two adjacent carbons by an alkylenedioxy radical.

**8.** The compounds of formula (I) as defined in claim 7 in which R represents a phenyl radical substituted by an alkyloxy radical containing up to 4 carbon atoms, by a halogen atom or simultaneously by these two substituents.

**9.** The compounds of formula (I) as defined in claim 8 in which R represents a phenyl radical substituted by a methoxy radical.

**10.** The compounds of formula (I) as defined in claim 8 in which R represents a phenyl radical substituted by a chlorine atom.

**11.** The compounds of formula (I) as defined in claim 8 in which R represents a phenyl radical substituted by a bromine atom.

**12.** The compounds of formula (I) as defined in claim 7 in which R represents a phenyl radical substituted by a trifluoromethyl radical.

**13.** The compounds of formula (I) as defined in claim 7 in which R represents a 3,4-methylenedioxyphenyl radical.

**14.** The compounds of formula (I) as defined in any one of claims 1 to 4 in which R represents a thiazolyl radical optionally substituted by a linear or branched alkyl radical containing up to 8 carbon atoms or by an optionally substituted phenyl radical.

**15.** The compounds of formula (I) as defined in claim 14 in which R represents a thiazolyl radical substituted by an isopropyl radical.

**16.** The compounds of formula (I) as defined in claim 14 in which R represents a thiazolyl radical substituted by a trifluoromethoxyphenyl radical.

**17.** The compounds of formula (I) as defined in any one of claims 1 to 16 in which the enol ether double bond is of E geometry.

**18.** The compounds of formula (I) as defined in claim 17 the names of which follow:
- methyl alpha-[(E)-methoxymethylene] 2-(4-methoxy phenyl) 7-benzofuranacetate
- methyl alpha-[(E)-methoxymethylene] 2-(3-methoxy phenyl) 7-benzofuranacetate
- methyl alpha-[(E)-methoxymethylene] 2-(3-chloro phenyl) 7-benzofuranacetate
- methyl alpha-[(E)-methoxymethylene] 2-(2-chloro phenyl) 7-benzofuranacetate
- methylene alpha-[(E)-methoxymethylene] 2-[4-bromo 3-(trifluoromethyl) phenyl] benzofuranacetate
- methylene alpha-[(E)-methoxymethylene] 2-[4-chloro (3-methoxy) phenyl] 7-benzofuranacetate
- methyl alpha-[(E)-methoxymethylene] 7-[2-[4-(2-isopropylthiazolyl) benzofuranyl] acetate
- methyl alpha-[(E)-methoxymethylene] 2-(3,4-methylenedioxy) phenyl 7-benzofuranacetate
- methyl alpha-[(E)-methoxymethylene] 2-butyl 7-benzofuranacetate
- methyl alpha-[(E)-methoxymethylene] 2-[2-[4-(trifluoromethoxy) phenyl] 4-thiazolyl] 7-benzofuranacetate,
- methyl alpha-[(E)-methoxymethylene] 2-[4-(2-trifluoromethyl) thiazolyl] 7-benzofuranacetate.

**19.** Preparation process for the compounds of formula (I) as defined in any one of claims 1 to 18, characterized in that a compound of formula (II):

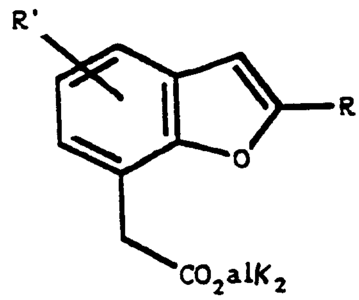

(II)

in which R, R' and $alk_2$ retain their meaning indicated in claim 1, is subjected to the action of a formylation agent in order to obtain the compound of formula (III):

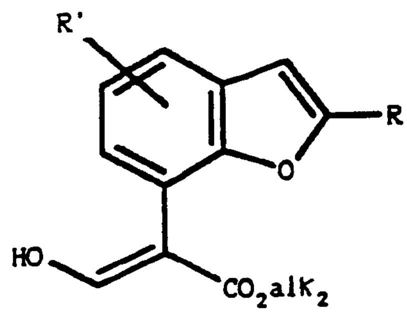

(III)

which is subjected to the action of an alkylation agent in order to obtain the compound of formula ($I_A$):

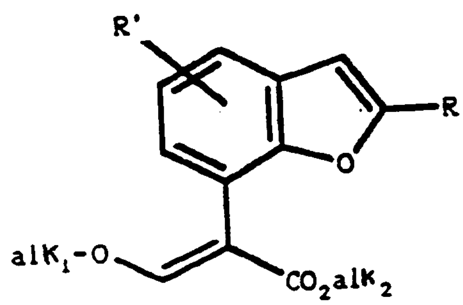

($I_A$)

in which $alk_1$ retains the meaning indicated in claim 1, which is subjected if desired to the action of an agent capable of replacing the oxygen atom by a sulphur atom in order to obtain the compound of formula ($I_B$):

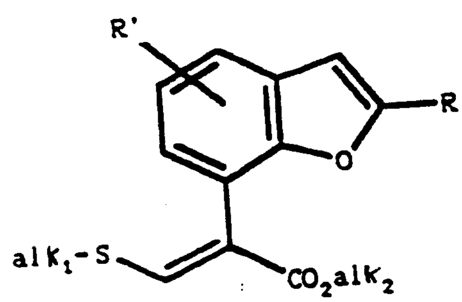

($I_B$)

**20.** The compounds of formulae (II) and (III) defined in claim 19, as new industrial products.

**21.** The pesticide compositions containing as active ingredient at least one compound of formula (I) as defined in any one of claims 1 to 18.

**22.** The insecticide compositions containing as active ingredient at least one compound of formula (I) as defined in any one of claims 1 to 18.

**23.** The acaricide compositions containing as active ingredient at least one compound of formula (I) as defined in any one of claims 1 to 18.

**24.** The fungicide compositions containing as active ingredient at least one compound of formula (I) as defined in any one of claims 1 to 18.

**Claims for the following Contracting State : ES**

**1.** Process for preparing the compounds of formula (I):

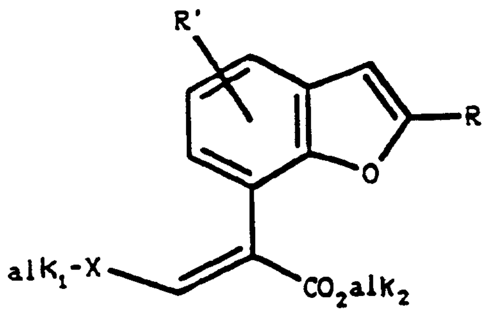

(I)

in which:

- R represents a hydrogen atom, an optionally substituted alkyl, alkenyl or alkynyl radical containing up to 8 carbon atoms, an optionally substituted cycloalkyl or cycloalkenyl radical containing up to 8 carbon atoms, an optionally substituted aryl radical containing up to 18 carbon atoms, an optionally substituted arylalkyl radical containing up to 24 carbon atoms, an optionally substituted benzoyl radical or an optionally substituted heterocyclic radical,
- $alk_1$ and $alk_2$, identical or different, represent an optionally substituted alkyl radical containing up to 8 carbon atoms,
- X represents an oxygen or sulphur atom,
- R' in any position on the phenyl nucleus represents a hydrogen atom or a halogen atom,

the geometry of the double bond being E or Z or an E and Z mixture, characterized in that a compound of formula (II):

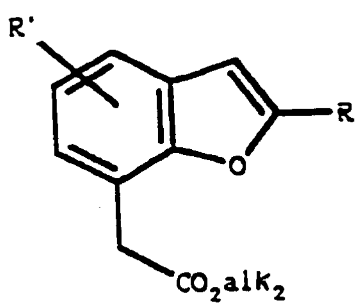

(II)

in which R, R' and $alk_2$ retain their meaning indicated previously, is subjected to the action of a formylation agent in order to obtain the compound of formula (III):

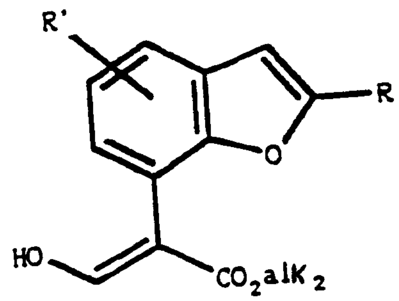

(III)

which is subjected to the action of an alkylation agent in order to obtain the compound of formula ($I_A$):

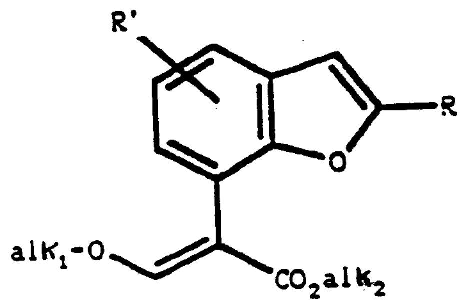

($I_A$)

in which $alk_1$ retains the meaning indicated previously, which is subjected if desired to the action of an agent capable of replacing the oxygen atom by a sulphur atom in order to obtain the compound of formula ($I_B$):

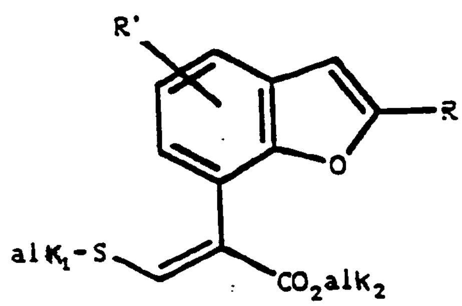

($I_B$)

**2.** Process according to claim 1, characterized in that a product of formula (II) is used at the start in which R' represents a hydrogen atom.

**3.** Process according to claim 1 or 2, characterized in that a product of formula (II) is used at the start in which $alk_2$ represents a methyl radical and in that a methylation agent is reacted on the product of formula (III) obtained.

**4.** Process according to any one of claims 1 to 3, characterized in that a product of formula (II) is used at the start in which R represents an alkyl radical containing up to 8 carbon atoms.

**5.** Process according to claim 4, characterized in that a product of formula (II) is used at the start in which R represents an n-butyl radical.

**6.** Process according to any one of claims 1 to 3, characterized in that a product of formula (II) is used at the start in which R represents a phenyl radical optionally substituted by one or more radicals chosen from the group constituted by halogen atoms, phenoxy, thiazolyl and alkyloxy radicals containing up to

6 carbon atoms and alkyl radicals substituted by one or more halogen atoms or R represents a phenyl radical substituted on two adjacent carbons by an alkylenedioxy radical.

**7.** Process according to claim 6, characterized in that a product of formula (II) is used at the start in which R represents a phenyl radical substituted by an alkyloxy radical containing up to 4 carbon atoms, by a halogen atom or simultaneously by these two substituents.

**8.** Process according to claim 7, characterized in that a product of formula (II) is used at the start in which R represents a phenyl radical substituted by a methoxy radical.

**9.** Process according to claim 6, characterized in that a product of formula (II) is used at the start in which R represents a phenyl radical substituted by a chlorine atom.

**10.** Process according to claim 6, characterized in that a product of formula (II) is used at the start in which R represents a phenyl radical substituted by a bromine atom.

**11.** Process according to claim 6, characterized in that a product of formula (II) is used at the start in which R represents a phenyl radical substituted by a trifluoromethyl radical.

**12.** Process according to claim 6, characterized in that a product of formula (II) is used at the start in which R represents a 3,4-methylenedioxyphenyl radical.

**13.** Process according to any one of claims 1 to 3, characterized in that a product of formula (II) is used at the start in which R represents a thiazolyl radical optionally substituted by a linear or branched alkyl radical containing up to 8 carbon atoms or by an optionally substituted phenyl radical.

**14.** Process according to claim 12, characterized in that a product of formula (II) is used at the start in which R represents a thiazolyl radical substituted by an isopropyl radical.

**15.** Process according to claim 12, characterized in that a product of formula (II) is used at the start in which R represents a thiazolyl radical substituted by a trifluoromethoxyphenyl radical.

**16.** Process according to any one of claims 1 to 15, characterized in that the product of formula (II) and the reagents are chosen in such a manner that one of the products of which the names follow is prepared:

- methyl alpha-[(E)-methoxymethylene] 2-(4-methoxy phenyl) 7-benzofuranacetate
- methyl alpha-[(E)-methoxymethylene] 2-(3-methoxy phenyl) 7-benzofuranacetate
- methyl alpha-[(E)-methoxymethylene] 2-(3-chloro phenyl) 7-benzofuranacetate
- methyl alpha-[(E)-methoxymethylene] 2-(2-chloro phenyl) 7-benzofuranacetate
- methylene alpha-[(E) methoxymethylene] 2-[4-bromo 3-(trifluoromethyl) phenyl] benzofuranacetate
- methylene alpha-[(E)-(methoxymethylene) 2-[4-chloro (3-methoxy) phenyl] 7-benzofuranacetate
- methyl alpha-[(E)-methoxymethylene] 7-[2-[4-(2-isopropylthiazolyl) benzofuranyl] acetate
- methyl alpha-[(E)-methoxymethylene] 2-(3,4-methylenedioxy) phenyl 7-benzofuranacetate
- methyl alpha-[(E)-methoxymethylene] 2-butyl 7-benzofuranacetate
- methyl alpha-[(E)-methoxymethylene] 2-[2-[4-(trifluoromethoxy) phenyl] 4-thiazolyl] 7-benzofuranacetate.

**Patentansprüche**
**Patentansprüche für folgende Vertragsstaaten : CH, DE, FR, GB, IT, LI, NL**

1. Verbindungen der Formel (I)

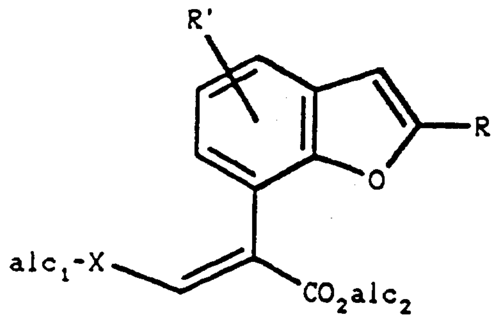

(I)

worin
R für ein Wasserstoffatom, einen Alkyl-, Alkenyl-oder Alkinylrest mit bis zu 8 Kohlenstoffatomen, welcher gegebenenfalls substituiert ist, einen Cycloalkyl- oder Cycloalkenylrest mit bis zu 8 Kohlenstoffatomen, welcher gegebenenfalls substituiert ist, einen Arylrest mit bis zu 18 Kohlenstoffatomen, welcher gegebenenfalls substituiert ist, einen Aralkylrest mit bis zu 24 Kohlenstoffatomen, welcher gegebenenfalls substituiert ist, einen Benzoylrest, welcher gegebenenfalls substituiert ist, oder einen heterocyclischen Rest, welcher gegebenenfalls substituiert ist, steht,
$alc_1$ und $alc_2$, identisch oder verschieden, einen Alkylrest mit bis zu 8 Kohlenstoffatomen, welcher gegebenenfalls substituiert ist, bedeuten,
X ein Sauerstoff- oder Schwefelatom wiedergibt,
R' in beliebiger Stellung an dem Phenylring für ein Wasserstoffatom oder ein Halogenatom steht, wobei die Geometrie der Doppelbindung die E- oder Z-Geometrie oder ein Gemisch von E und Z darstellt.

2. Verbindungen der Formel (I), wie in Anspruch 1 definiert, worin X für ein Sauerstoffatom steht.

3. Verbindungen der Formel (I),wie in Anspruch 1 oder 2 definiert, worin R' für ein Wasserstoffatom steht.

4. Verbindungen der Formel (I), wie in einem der Ansprüche 1 bis 3 definiert, worin $alc_1$ und $alc_2$ jeweils für einen Methylrest stehen.

5. Verbindungen der Formel (I), wie in einem der Ansprüche 1 bis 4 definiert, worin R für einen Alkylrest mit bis zu 8 Kohlenstoffatomen steht.

6. Verbindungen der Formel (I), wie in Anspruch 5 definiert, worin R für einen n-Butylrest steht.

7. Verbindungen der Formel (I), wie in einem der Ansprüche 1 bis 4 definiert, worin R für einen Phenylrest steht, welcher gegebenenfalls durch einen oder mehrere Reste, ausgewählt unter den Halogenatomen, den Phenoxy-, Thiazolyl- und Alkoxyresten mit bis zu 6 Kohlenstoffatomen und Alkyl, substituiert durch einen oder mehrere Halogenatome, substituiert ist, oder R für einen Phenylrest steht, welcher an zwei benachbarten Kohlenstoffatomen durch einen Alkylendioxyrest substituiert ist.

8. Verbindungen der Formel (I), wie in Anspruch 7 definiert, worin R für einen Phenylrest steht, welcher durch einen Alkoxyrest mit bis zu 4 Kohlenstoffatomen, durch ein Halogenatom oder gleichzeitig durch diese beiden Substituenten substituiert ist.

9. Verbindungen der Formel (I), wie in Anspruch 8 definiert, worin R für einen Phenylrest steht, welcher durch einen Methoxyrest substituiert ist.

**10.** Verbindungen der Formel (I), wie in Anspruch 8 definiert, worin R für einen Phenylrest steht, welcher durch ein Chloratom substituiert ist.

**11.** Verbindungen der Formel (I), wie in Anspruch 8 definiert, worin R für einen Phenylrest steht, welcher durch ein Bromatom substituiert ist.

**12.** Verbindungen der Formel (I), wie in Anspruch 7 definiert, worin R für einen Phenylrest steht, welcher durch einen Trifluormethylrest substituiert ist.

**13.** Verbindungen der Formel (I), wie in Anspruch 7 definiert, worin R für einen 3,4-Methylendioxyphenylrest steht.

**14.** Verbindungen der Formel (I), wie in einem der Ansprüche 1 bis 4 definiert, worin R für einen Thiazolylrest steht, welcher gegebenenfalls durch einen linearen oder verzweigten Alkylrest mit bis zu 8 Kohlenstoffatomen oder durch einen gegebenenfalls substituierten Phenylrest substituiert ist.

**15.** Verbindungen der Formel (I), wie in Anspruch 14 definiert, worin R für einen Thiazolylrest steht, welcher durch einen Isopropylrest substituiert ist.

**16.** Verbindungen der Formel (I), wie in Anspruch 14 definiert, worin R für einen Thiazolylrest steht, der durch einen Trifluormethoxyphenylrest substituiert ist.

**17.** Verbindungen der Formel (I), wie in einem der Ansprüche 1 bis 16 definiert, worin die Enolether-Doppelbindung die E-Geometrie besitzt.

**18.** Verbindungen der Formel (I), wie in Anspruch 17 definiert, mit den folgenden Bezeichnungen:
α-[(E)-Methoxymethylen]-2-(4-methoxyphenyl)-7-benzofuran-methylacetat
α-[(E)-Methoxymethylen]-2-(3-methoxyphenyl)-7-benzofuran-methylacetat
α-[(E)-Methoxymethylen]-2-(3-chlorphenyl)-7-benzofuran-methylacetat
α-[(E)-Methoxymethylen]-2-(2-chlorphenyl)-7-benzofuran-methylacetat
α-[(E)-Methoxymethylen]-2-[4-brom-3-(trifluormethyl)-phenyl]-benzofuran-methylenacetat
α-[(E)-Methoxymethylen]-2-[4-chlor-(3-methoxy)-phenyl]-7-benzofuran-methylenacetat
α-[(E)-Methoxymethylen]-7-[2-[4-(2-isopropylthiazolyl)-benzofuranyl]-methylacetat
α-[(E)-Methoxymethylen]-2-(3,4-methylendioxy)-phenyl-7-benzofuran-methylacetat
α-[(E)-Methoxymethylen]-2-butyl-7-benzofuranmethylacetat
α-[(E)-Methoxymethylen]-2-[2-[4-(trifluormethoxy)-phenyl]-4-thiazolyl]-7-benzofuran-methylacetat
α-[(E)-Methoxymethylen]-2-[4-(2-trifluormethyl)-thiazolyl]-7-benzofuran-methylacetat.

**19.** Verfahren zur Herstellung der Verbindungen der Formel (I), wie in einem der Ansprüche 1 bis 18 definiert, dadurch gekennzeichnet, daß man eine Verbindung der Formel (II)

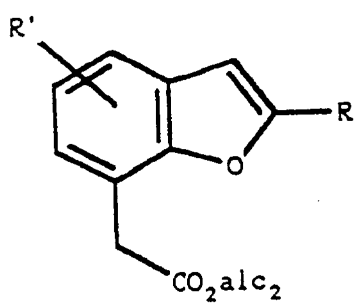

(II)

worin R, R' und $alc_2$ die in Anspruch 1 angegebene Bedeutung besitzen, der Einwirkung eines Formylierungsmittels unterzieht, um zu der Verbindung der Formel (III)

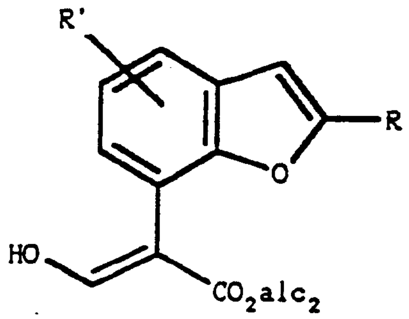

(III)

zu gelangan, welche man der Einwirkung eines Alkylierungsmittels unterzieht, um zu der Verbindung der Formel ($I_A$)

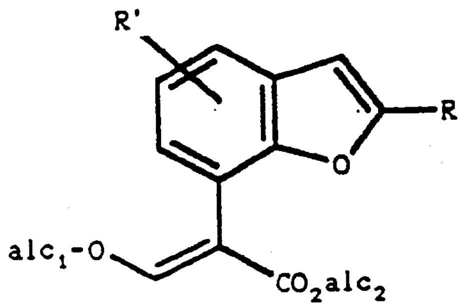

($I_A$)

zu gelangen, worin $alc_1$ die in Anspruch 1 angegebene Bedeutung besitzt, welche man gewünschtenfalls der Einwirkung eines Mittels, das befähigt ist, das Sauerstoffatom durch ein Schwefelatom zu ersetzen, unterzieht, um zu der Verbindung der Formel ($I_B$)

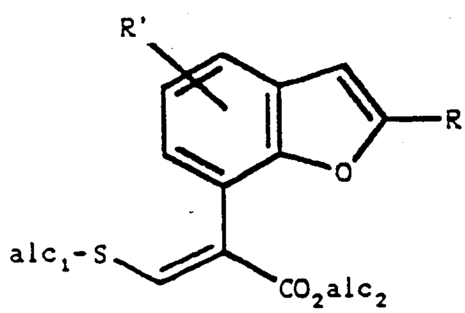

($I_B$)

zu gelangen.

**20.** Verbindungen der Formel (II) und (III), wie in Anspruch 19 definiert, als neue industrielle Produkte.

**21.** Pestizide Zusammensetzungen, enthaltend als Wirkstoff zumindest eine Verbindung der Formel (I), wie in einem der Ansprüche 1 bis 18 definiert.

**22.** Insektizide Zusammensetzungen, enthaltend als Wirkstoff zumindest eine Verbindung der Formel (I), wie in einem der Ansprüche 1 bis 18 definiert.

**23.** Akarizide Zusammensetzungen, enthaltend als Wirkstoff zumindest eine Verbindung der Formel (I), wie in einem der Ansprüche 1 bis 18 definiert.

**24.** Fungizide Zusammensetzungen, enthaltend als Wirkstoff zumindest eine Verbindung der Formel (I), wie in einem der Ansprüche 1 bis 18 definiert.

**Patentansprüche für folgenden Vertragsstaat : ES**

1. Verfahren zur Herstellung der Verbindungen der Formel (I)

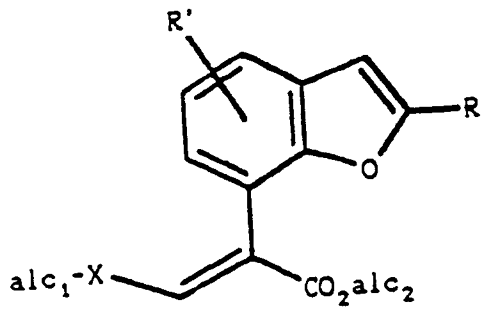

(I)

worin

R für ein Wasserstoffatom, einen Alkyl-, Alkenyl-oder Alkinylrest mit bis zu 8 Kohlenstoffatomen, welcher gegebenenfalls substituiert ist, einen Cycloalkyl- oder Cycloalkenylrest mit bis zu 8 Kohlenstoffatomen, welcher gegebenenfalls substituiert ist, einen Arylrest mit bis zu 18 Kohlenstoffatomen, welcher gegebenenfalls substituiert ist, einen Aralkylrest mit bis zu 24 Kohlenstoffatomen, welcher gegebenenfalls substituiert ist, einen Benzoylrest, welcher gegebenenfalls substituiert ist, oder einen heterocyclischen Rest, welcher gegebenenfalls substituiert ist, steht,

$alc_1$ und $alc_2$, identisch oder verschieden, einen Alkylrest mit bis zu 8 Kohlenstoffatomen, welcher gegebenenfalls substituiert ist, bedeuten,

X ein Sauerstoff- oder Schwefelatom bedeutet,

R' in beliebiger Stellung an dem Phenylring für ein Wasserstoffatom oder ein Halogenatom steht, wobei die Geometrie der Doppelbindung die E- oder Z-Geometrie oder ein Gemisch von E und Z darstellt,

dadurch gekennzeichnet, daß man eine Verbindung der Formel (II)

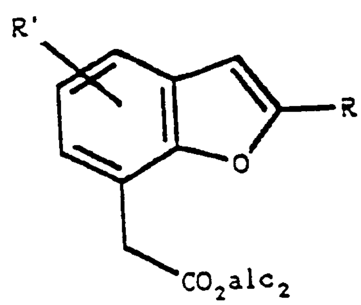

(II)

worin R, R' und $alc_2$ die vorstehend angegebene Bedeutung besitzen, der Einwirkung eines Formylierungsmittels unterzieht, um zu der Verbindung der Formel (III)

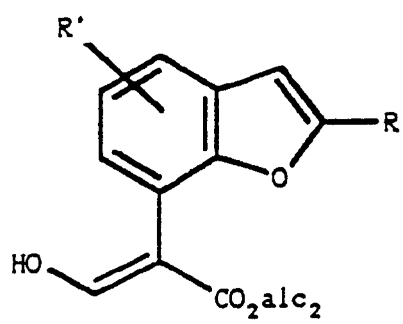

(III)

zu gelangen, die man der Einwirkung eines Alkylierungsmittels unterzieht, um zu der Verbindung der Formel ($I_A$)

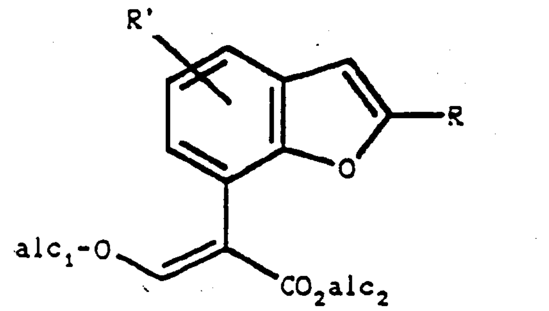

($I_A$)

zu gelangen, worin $alc_1$ die vorstehend angegebene Bedeutung besitzt, welche man gewünschtenfalls der Einwirkung eines Mittels, das befähigt ist, das Sauerstoffatom durch ein Schwefelatom zu ersetzen, unterzieht, um zu der Verbindung der Formel ($I_B$)

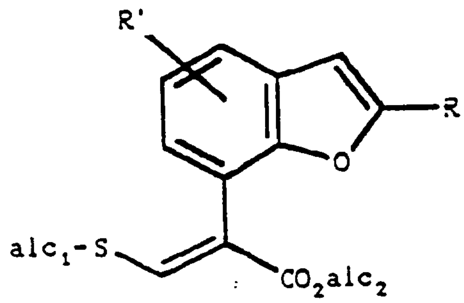

($I_B$)

zu gelangen.

2. Verfahren gemäß Anspruch 1, dadurch gekennzeichnet, daß man von einem Produkt der Formel (II) ausgeht, worin R' für ein Wasserstoffatom steht.

3. Verfahren gemäß Anspruch 1 oder 2, dadurch gekennzeichnet, daß man von einem Produkt der Formel (II) ausgeht, worin $alc_2$ für einen Methylrest steht,und daß man ein Methylierungsmittel mit dem erhaltenen Produkt der Formel (III) umsetzt.

4. Verfahren gemäß einem der Ansprüche 1 bis 3, dadurch gekennzeichnet, daß man von einem Produkt der Formel (II) ausgeht, worin R für einen Alkylrest mit bis zu 8 Kohlenstoffatomen steht.

5. Verfahren gemäß Anspruch 4, dadurch gekennzeichnet, daß man von einem Produkt der Formel (II) ausgeht, worin R für einen n-Butylrest steht.

6. Verfahren gemäß einem der Ansprüche 1 bis 3, dadurch gekennzeichnet, daß man von einem Produkt der Formel (II) ausgeht, worin R für einen Phenylrest steht, der gegebenenfalls durch einen oder mehrere Reste substituiert ist, ausgewählt unter den Halogenatomen, den Phenoxy-, Thiazolyl- und Alkoxyresten mit bis zu 6 Kohlenstoffatomen und Alkyl, substituiert durch ein oder mehrere Halogenatome, oder R für einen Phenylrest steht, der an zwei benachbarten Kohlenstoffatomen durch einen Alkylendioxyrest substituiert ist.

7. Verfahren gemäß Anspruch 6, dadurch gekennzeichnet, daß man von einem Produkt der Formel (II) ausgeht, worin R für einen Phenylrest steht, welcher durch einen Alkoxyrest mit bis zu 4 Kohlenstoffatomen, durch ein Halogenatom oder gleichzeitig durch diese beiden Substituenten substituiert ist.

**8.** Verfahren gemäß Anspruch 7, dadurch gekennzeichnet, daß man von einem Produkt der Formel (II) ausgeht, worin R für einen Phenylrest steht, welcher durch einen Methoxyrest substituiert ist.

**9.** Verfahren gemäß Anspruch 6, dadurch gekennzeichnet, daß man von einem Produkt der Formel (II) ausgeht, worin R für einen Phenylrest steht, welcher durch ein Chloratom substituiert ist.

**10.** Verfahren gemäß Anspruch 6, dadurch gekennzeichnet, daß man von einem Produkt der Formel (II) ausgeht, worin R für einen Phenylrest steht, welcher durch ein Bromatom substituiert ist.

**11.** Verfahren gemäß Anspruch 6, dadurch gekennzeichnet, daß man von einem Produkt der Formel (II) ausgeht, worin R für einen Phenylrest steht, der durch einen Trifluormethylrest substituiert ist.

**12.** Verfahren gemäß Anspruch 6, dadurch gekennzeichnet, daß man von einem Produkt der Formel (II) ausgeht, worin R für einen 3,4-Methylendioxyphenylrest steht.

**13.** Verfahren gemäß einem der Ansprüche 1 bis 3, dadurch gekennzeichnet, daß man von einem Produkt der Formel (II) ausgeht, worin R für einen Thiazolylrest steht, welcher gegebenenfalls durch einen linearen oder verzweigten Alkylrest mit bis zu 8 Kohlenstoffatomen oder durch einen gegebenenfalls substituierten Phenylrest substituiert ist.

**14.** Verfahren gemäß Anspruch 12, dadurch gekennzeichnet, daß man von einem Produkt der Formel (II) ausgeht, worin R für einen Thiazolylrest steht, welcher durch einen Isopropylrest substituiert ist.

**15.** Verfahren gemäß Anspruch 12, dadurch gekennzeichnet, daß man von einem Produkt der Formel (II) ausgeht, worin R für einen Thiazolylrest steht, welcher durch einen Trifluormethoxyphenylrest substituiert ist.

**16.** Verfahren gemäß einem der Ansprüche 1 bis 15, dadurch gekennzeichnet, daß man das Produkt der Formel (II) und die Reagentien derart auswählt, daß man eines der Produkte mit den folgenden Bezeichnungen herstellt:

α-[(E)-Methoxymethylen]-2-(4-methoxyphenyl)-7-benzofuran-methylacetat
α-[(E)-Methoxymethylen]-2-(3-methoxyphenyl)-7-benzofuran-methylacetat
α-[(E)-Methoxymethylen]-2-(3-chlorphenyl)-7-benzofuran-methylacetat
α-[(E)-Methoxymethylen]-2-(2-chlorphenyl)-7-benzofuran-methylacetat
α-[(E)-Methoxymethylen]-2-[4-brom-3-(trifluormethyl)-phenyl]-benzofuran-methylenacetat
α-[(E)-Methoxymethylen]-2-[4-chlor-(3-methoxy)-phenyl]-7-benzofuran-methylenacetat
α-[(E)-Methoxymethylen]-7-[2-[4-(2-isopropylthiazolyl)-benzofuranyl]-methylacetat
α-[(E)-Methoxymethylen]-2-(3,4-methylendioxy)-phenyl-7-benzofuran-methylacetat
α-[(E)-Methoxymethylen]-2-butyl-7-benzofuran-methylacetat
α-[(E)-Methoxymethylen]-2-[2-[4-(trifluormethoxy)-phenyl]-4-thiazolyl]-7-benzofuran-methylacetat.